# EUROPEAN PATENT APPLICATION

(11) **EP 1 283 037 A1**
(43) Date of publication of application: **12.02.2003**
(21) Application number: 01919859.7
(22) Date of filing: 10.04.2001
(51) Int. Cl.: A61K 31/12, A61K 31/216, A61K 31/352, A61K 31/7024, A61P 25/00, A61P 1/16, A61P 43/00, A23L 1/30

(54) **REMEDIES**

(30) Priority: 11.04.2000 JP 2000109983; 06.10.2000 JP 2000308524; 06.10.2000 JP 2000308525
(71) Applicant: TAKARA BIO INC., Otsu-shi, Shiga 520-2193 (JP)
(72) Inventor: OHNOGI, Hiromu, Muko-shi, Kyoto 617-0002 (JP); KOBAYASHI, Eiji, Otsu-shi, Shiga 520-2153 (JP); NISHIMURA, Kaori, Otsu-shi, Shiga 520-0853 (JP); SHIRAGA, Masahiro, Otsu-shi, Shig a 520-2134 (JP); LI, Tuo-Ping, c/o Central Res. Lab., Otsu-shi, Shiga 520-2193 (JP); SAGAWA, Hiroaki, Kusatsu-shi, Shiga 525-0025 (JP); KATO, Ikunoshin, Uji-shi, Kyoto 611-0028 (JP)
(74) Representative: VOSSIUS & PARTNER
(86) International application number: JP0103075
(87) International publication number: WO01076580

(57) **Abstract**

The present invention provides a therapeutic agent or prophylactic agent for a disease requiring enhancement for growth factor production and a food, beverage or feed for enhancing growth factor production, each comprising as an effective ingredient: polyphenols, derivatives thereof, and/or salts thereof; or a composition obtained by subjecting polyphenols, derivatives thereof, and/or salts thereof to: (i) a mixing treatment with a metal, a metal salt and a metal ion, or (ii) an oxidation treatment.

## Description

### TECHNICAL FIELD

The present invention relates to a medicament, food beverage or feed, comprising as an effective ingredient a polyphenol, a derivative thereof and/or a salt thereof.

### BACKGROUND ART

Polyphenol is a generic term of substances having several or more phenolic hydroxyl groups in its molecule, many of which have been known to be derived from plants. In a broad sense, the polyphenol includes flavonoids, chlorogenic acid, caffeic acid, dicaffeoylquinic acid, gallic acid, proanthocyanin, gallotannin, and the like. Also, the flavonoids are further classified according to their basic backbones, and can be classified into flavones, flavonols, flavanones, anthocyanidins, chalcones, isoflavones, flavanols, flavanonols, and the like. The flavonols include, for instance, myricetin, quercetin, and the like. The flavanols include catechins, for instance, epigallocatechin gallate, and the like. The flavanones include isoxanthohumol, and the like. The chalcones include xanthohumol B, xanthohumol D and the like.

Although antioxidating activity has been primarily known as physiological activities of the polyphenol, the enhancing activity for growth factor production therefor has not yet been known.

Nerve cells play a principal role for sustaining psychoactivities of human being such as intellectual functions, memory, emotions and behaviors. It has been thought that the differentiation, survival and exhibition of functions of the nerve cells which are the foundations of these psychoactivities need a neurotrophic factor specific for each nerve cell. Among the neurotrophic factors, one of which existence and function have been firstly elucidated is a nerve growth factor (hereinafter simply referred to as "NGF"), and currently, there have been found a brain-derived-neurotrophic factor, neurotrophin-3, neurotrophin-4/5, and the like.

NGF is a neurotrophic factor of a large cellular cholinergic nerve cell of basal portion of the forebrain, so that its association with Alzheimer's dementia has been remarked [*Pharmacia*, Vol.22, No.2, 147-151 (1986), *Ronen Seishin Igaku (Senile Psychiatry*), Vol.3, No.6, 751-758 (1986)]. Alzheimer's dementia refers to a disease that gives a pathological finding such as senile plaque or Alzheimer's fibrillar changes, which are accompanied by a clinical picture such as developmental disability, manic state, tonic seizures of lower limbs, or epileptic seizure, and is one disease of senile dementia. The Alzheimer's dementia tends to be increasing in recent aging society, so that a larger societal interest has been drawn thereto. However, there has not yet been found a method for ameliorating or treating such symptoms.

In the brain of a patient with Alzheimer's dementia, there has been found a dramatic denaturation and a drastic lowering of the activity of choline acetyl transferase (CAT) in the basal portion of the forebrain centering about Meynert's basal nuclei [*Annu. Rev. Neurosci*., Vol.3, 77 (1980)]. In the studies of a rat brain in 1985, there has been elucidated that NGF is a neurotrophic factor at this site of the brain [*EMBO J*., Vol.4, 1389 (1985)], so that the association of NGF with this disease has been remarked. In addition, there have been elucidated that in the striate body of the brain of a patient with Huntington's chorea, there are remarkable detachment of GABAergic nerve cell as well as detachment of cholinergic nerve cell, so that NGF also acts on the endogenous cholinergic nerve cell of the striate body [*Science,* Vol.234, 1341 (1986)], addressing a possibility that this disease is associated with NGF. The effects of NGF have been studied with an animal such as a rat which can serve as a model for various nerve diseases. There has been reported that the degeneration of the nerve cell can be stopped in a rat if NGF is intracerebrally administered before the degeneration becomes remarkable, and that the lowering of CAT activity is also prevented [*J. Neurosci.,* Vol.6, 2155 (1986), *Brain Res.,* Vol.293, 305 (1985), *Science,* Vol.235, 214 (1986), *Proc. Natl. Acad. Sci. USA*, Vol.83, 9231 (1986)]. Also, it has been proven that NGF is biosynthesized in the peripheral sympathetic nerve-dominant tissues and in the brain, and that each of fibroblasts or astroglia which are interstitial cells for peripheral tissues or brain tissues plays an important role for the NGF biosynthesis [*J. Biol. Chem*., Vol.259, 1259 (1984), *Biochem. Biophys. Res. Commun.,* Vol.136, 57 (1986)]. In addition, it has been elucidated that antigenicity, molecular weight, isoelectric point and biological activity of the fibroblast-producing or astroglia-producing NGF are the same as NGF of conventionally well studied submandibular gland. At the same time, it has been found that a catecholamine such as norepinephrine, epinephrine or dopamine shows enhancing action for NGF production by a test of adding various neurotransmitters to a culture medium of fibroblasts (L-M cells) and astroglia [*J. Biol. Chem.*, Vol.201, 6039 (1986)].

There has been expected that NGF can be used as a therapeutic agent for stopping degeneration in a nerve disease in which a site at which NGF acts as a neurotrophic factor is degenerated. In addition, once the cranial nerve cells are degenerated by cebrovascular disorders, cerebral tumor, cerebral apicitus, nerve degenerative disease caused by head injury, intoxication with an anesthetic, or the like, the degenerated cranial nerve cells would never recover during the life time, whereby various disorders such as emotional disorders and behavioral abnormality are consequently caused in addition to lowering in the intellectual functions and memory disabilities. On the other hand, nerve fiber shows plasticity, that is, when the nerve fiber is damaged, budding takes place from its surrounding healthy fibers, so that a new synapsis is formed in place of the damaged synapsis. Therefore, it has been expected that NGF can be used as a therapeutic agent for promoting restoration and regeneration of nerve functions at this stage.

However, when NGF is applied to a treatment of various nerve diseases, NGF must reach in very close vicinity of nerve cell that requires NGF, and NGF must be transmitted to lesion site of the cranial cell in a case of a disease in the central nervous system. However, NGF cannot be transmitted into the brain through the blood system. This is because the vascular endothelial cells in the brain are bound to each other by adhesion bonding (referred to as brain blood barrier), so that there is a limitation in the transport of a substance other than water, gas or an oil-soluble substance from blood to a brain tissue, whereby a protein (including NGF), which is polymeric substance, cannot pass through the brain blood barrier. There is a too large risk involved in the introduction of NGF directly into the brain by a surgical means, even if the introduction is conducted by the current techniques.

On the other hand, there has been developed a substance for enhancing NGF production, not a direct administration of NGF. Most of the compounds, however, have various problems such that the compounds have strong toxicity, or the compounds have effective concentration very closely approximating concentration at which toxicity is shown, or the compounds have severe adverse actions against nervous system such as nerve excitation action. Therefore, these compounds have not yet been actually used.

A liver subjected to partial hepatectomy quickly regenerates and regains its original size. Although the substance of the factor for hepatic regeneration has been unknown for many years, hepatocyte growth factor (HGF) has been found in plasma of a patient suffering from fulminant hepatitis, and isolated and purified from the plasma of this patient (Gohda, E. et al.: *J. Clin. Invest.,* **88** 414-419, 1988). Further, human HGF cDNA has been also cloned, and the primary structure for HGF has been also elucidated (Miyakawa, K. et al.: *Biochem. Biophys. Res. Commun.,* **163** 967-973, 1989). In addition, it has been elucidated that scatter factor (SF) for facilitating motility of cells, and a tumor cell disorder factor, tumor cytotoxic factor (TCF) are identical substances to HGF (Weidner, K. M. et al.: *Proc. Natl. Acad. Sci. USA*, **88** 7001-7005, 1991; Shima, N. et al.: *Biochem. Biophys. Res. Commun.*, **180** 1151-1158, 1991).

HGF accelerates growth of many of epithelial cells, such as changioepithelial cells, renal tubule epithelial cells, and gastric mucosa cells, as well as hepatocytes, and induces morphological formations as seen in facilitation of motility of epithelial cells, vascularization or luminal formation of epithelial cells, so that HGF is a multi-functional active substance exhibiting a wide variety of physiological activity. In other words, in various organs, HGF induces morphological formations such as proliferation acceleration and facilitation of motility of epithelial cells, or vascularization during the recovery of the disorder of the organ, and the like.

HGF exhibits growing action for hepatocytes, accelerating action for protein synthesis, ameliorating action for cholestasia, and further preventing action for renal disorder caused by drugs and the like. mRNA of HGF is synthesized even in the brain, the kidney, the lungs, and the like. HGF is a mesoblast growth factor that accelerates growth of many of epithelial cells, such as changioepithelial cells, renal tubule epithelial cells, and gastric mucosa cells. In addition, it induces morphological formations such as proliferation acceleration and facilitation of motility of epithelial cells, or vascularization during the recovery of the disorder, so that HGF is a multi-functional active substance exhibiting a wide variety of physiological activity. Further, HGF also has actions for protection, proliferation acceleration, and recovery of disorder of nerve cells, and the like. Therefore, by enhancing the production of HGF, it has been expected to treat or prevent hepatic disorders such as hepatitis, severe hepatitis, fulminant hepatitis, cirrhosis, and cholestasia in the liver, renal disorders caused by drugs and the like, gastrointestinal disorders, vascular disorders, chronic nephritis, pneumonia, wound, diabetes, cancer, and the like.

Since HGF has the various actions mentioned above, the HGF itself is expected to be used as a therapeutic agent for hepatic disorders such as hepatitis, severe hepatitis, fulminant hepatitis, cirrhosis, and cholestasia in the liver, renal disorders caused by drugs and the like, gastrointestinal disorders, vascular disorders, chronic nephritis, pneumonia, wound, diabetes, cancer, and the like. However, the HGF itself has not yet been used as a therapeutic agent for actual use. Further, although a method of introducing a gene of HGF by gene therapy has been tried, it is far from being actually used because of adverse actions resulting from HGF actions caused in an unnecessary timing and location. As described above, if HGF can be arbitrarily enhanced without being externally administered, HGF is thought to be effective for treatment and prophylaxis of a disease requiring enhancement for HGF production, such as hepatic disorders such as hepatitis, severe hepatitis, fulminant hepatitis, cirrhosis, and cholestasia in the liver, renal disorders caused by drugs and the like, gastrointestinal disorders, vascular disorders, chronic nephritis, pneumonia, wound, diabetes, and cancer. However, this has not yet been actually used.

As described above, although it is thought that various diseases associated with growth factor can be treated or prevented by enhancing the growth factor, there have not been known substances, means, and the like capable of appropriately enhancing growth factor production as desired without showing toxicity or adverse actions.

### DISCLOSURE OF INVENTION

As a result of intensive studies, the present inventors have found that a polyphenol, a derivative thereof or a salt thereof has enhancing action for growth factor production. The present invention has been perfected thereby.

Concretely, the present invention relates to:
[1] a therapeutic agent or prophylactic agent for a disease requiring enhancement for growth factor production, characterized in that the agent comprises as an effective ingredient:
   (i) at least one kind selected from the group consisting of polyphenols, derivatives thereof, and salts thereof; or
   (ii) a composition obtained by subjecting at least one kind selected from the group consisting of polyphenols, derivatives thereof, and salts thereof to:
      (A) a mixing treatment with at least one kind selected from the group consisting of a metal, a metal salt and a metal ion, or
      (B) an oxidation treatment;
[2] the therapeutic agent or prophylactic agent according to item [1] above, wherein the polyphenol is at least one kind selected from the group consisting of flavonoids, gallic acid, chlorogenic acid, cryptochlorogenic acid, neochlorogenic acid, caffeic acid and dicaffeoylquinic acid;
[3] the therapeutic agent or prophylactic agent according to item [2] above, wherein the flavonoid is at least one kind selected from the group consisting of flavonols, flavanones, chalcones and flavanols;
[4] the therapeutic agent or prophylactic agent according to item [3] above, wherein the flavonol is myricetin and/or quercetin, the flavanone is isoxanthohumol, the chalcone is xanthohumol B and/or xanthohumol D, and the flavanol is epigallocatechin gallate;
[5] the therapeutic agent or prophylactic agent according to any one of items [1] to [4] above, wherein the derivative of a polyphenol is a carboxylic acid ester of a polyphenol and/or a glycoside of a polyphenol;
[6] the therapeutic agent or prophylactic agent according to item [5] above, wherein the carboxylic acid ester of a polyphenol is caffeic acid methyl ester and/or caffeic acid ethyl ester, and the glycoside of a polyphenol is isoorientin;
[7] the therapeutic agent or prophylactic agent according to any one of items [1] to [6] above, wherein the metal is at least one selected from the group consisting of iron, manganese, magnesium, copper, zinc, silver, gold, aluminum, calcium, nickel and cobalt, the metal salt is a salt containing the above-mentioned metal, and the metal ion is an ion of the above-mentioned metal;
[8] the therapeutic agent or prophylactic agent according to any one of items [1] to [7] above, wherein the growth factor is a hepatocyte growth factor or a nerve growth factor;
[9] a food, beverage or feed for enhancing growth factor production, characterized in that the food, beverage or feed comprises as an effective ingredient:
   (i) at least one kind selected from the group consisting of polyphenols, derivatives thereof, and salts thereof; or
   (ii) a composition obtained by subjecting at least one kind selected from the group consisting of polyphenols, derivatives thereof, and salts thereof to:
      (A) a mixing treatment with at least one kind selected from the group consisting of a metal, a metal salt and a metal ion, or
      (B) an oxidation treatment;
[10] the food, beverage or feed according to item [9] above, wherein the polyphenol is at least one kind selected from the group consisting of flavonoids, gallic acid, chlorogenic acid, cryptochlorogenic acid, neochlorogenic acid, caffeic acid and dicaffeoylquinic acid;
[11] the food, beverage or feed according to item [10] above, wherein the flavonoid is at least one kind selected from the group consisting of flavonols, flavanones, chalcones and flavanols;
[12] the food, beverage or feed according to item [11] above, wherein the flavonol is myricetin and/or quercetin, the flavanone is isoxanthohumol, the chalcone is xanthohumol B and/or xanthohumol D, and the flavanol is epigallocatechin gallate;
[13] the food, beverage or feed according to any one of items [9] to [12] above, wherein the derivative of a polyphenol is a carboxylic acid ester of a polyphenol and/or a glycoside of a polyphenol;
[14] the food, beverage or feed according to item [13] above, wherein the carboxylic acid ester of a polyphenol is caffeic acid methyl ester and/or caffeic acid ethyl ester, and the glycoside of a polyphenol is isoorientin;
[15] the food, beverage or feed according to any one of items [9] to [14] above, wherein the metal is at least one selected from the group consisting of iron, manganese, magnesium, copper, zinc, silver, gold, aluminum, calcium, nickel and cobalt, the metal salt is a salt containing the above-mentioned metal, and the metal ion is an ion of the above-mentioned metal.
[16] the food, beverage or feed according to any one of items [9] to [15] above, wherein the growth factor is a hepatocyte growth factor or a nerve growth factor.
[17] a composition obtained by subjecting at least one kind selected from the group consisting of polyphenols, derivatives thereof, and salts thereof to:
   (A) a mixing treatment with at least one kind selected from the group consisting of a metal, a metal salt and a metal ion, or
   (B) an oxidation treatment;
[18] the composition according to item [17] above, wherein the polyphenol is chlorogenic acid and/or caffeic acid, and the derivative of a polyphenol is a carboxylic acid ester of the above-mentioned polyphenol and/or a glycoside of the above-mentioned polyphenol; and
[19] the composition according to item [17] or [18] above, wherein the metal is at least one selected from the group consisting of iron, manganese, magnesium, copper, zinc, silver, gold, aluminum, calcium, nickel and cobalt, the metal salt is a salt containing the above-mentioned metal, and the metal ion is an ion of the above-mentioned metal;
[20] the composition according to any one of items [17] to [19] above, which has enhancing action for growth factor production; and
[21] the composition according to item [20] above, wherein the growth factor is a hepatocyte growth factor or a nerve growth factor.

### BRIEF DESCRIPTION OF THE DRAWINGS

Figure 1 is MS spectrum of the fraction 18-3 derived from *Angelica keiskei* koidz.
Figure 2 is ¹H-NMR spectrum of the fraction 18-3 derived from *Angelica keiskei* koidz.
Figure 3 is MS spectrum of the fraction 28 derived from *Angelica keiskei* koidz.
Figure 4 is ¹H-NMR spectrum of the fraction 28 derived from *Angelica keiskei* koidz.
Figure 5 is MS spectrum of the fraction A-5 derived from xanthohumol fraction.
Figure 6 is ¹H-NMR spectrum of the fraction A-5 derived from xanthohumol fraction.

### BEST MODE FOR CARRYING OUT THE INVENTION

The effective ingredient according to the present invention is (i) at least one kind selected from the group consisting of polyphenols, derivatives thereof, and salts thereof (hereinafter referred to as "polyphenols and the like" in some cases); or (ii) a composition obtained by subjecting at least one kind selected from the group consisting of polyphenols, derivatives thereof, and salts thereof to (A) a mixing treatment with at least one kind selected from the group consisting of a metal, a metal salt and a metal ion, or (B) an oxidation treatment. The enhancing action for growth factor production of the effective ingredient is exhibited by the polyphenol, a derivative thereof, or a salt thereof, and these effective ingredients can be conveniently prepared and are very useful in wide range of fields such as medicaments and foods due to their various physiological functions. In addition, the enhancing action for growth factor production of the polyphenols and the like is enhanced by subjecting the polyphenols and the like to a treatment of (A) or (B) mentioned above. Therefore, the above-mentioned composition is more preferable one as the effective ingredient. The above-mentioned enhancing action for growth factor production of the polyphenols and the like and the enhancement of the above-mentioned action by the above-mentioned treatment of the polyphenols and the like have been found for the first time in the present invention.

In the present specification, the term "enhancing action for growth factor production" and "enhancing activity for growth factor production" refer to enhancement for growth factor production and a function for enhancing growth factor production, respectively, and are not intended to particularly strictly distinguish in their meaning. In addition, the term "enhance" encompasses an embodiment in which the amount of the desired substance is increased after the action as compared to that before the action of the effective ingredient according to the present invention, as well as an embodiment in which the desired substance is produced by the action of the effective ingredient according to the present invention (induce). In addition, in the present specification, any of the substances listed as the effective ingredients can be used alone or in admixture of two or more kinds in the present invention.

The polyphenols used in the present invention are not particularly limited, as long as they have enhancing action for growth factor production. For instance, there can be used any of those classified as flavonoids such as flavonols, chalcones, isoflavones, anthocyanins, flavanols, flavanones, flavones, aurones, and flavanonols; and those classified as non-flavonoids such as gallic acid, chlorogenic acid, cryptochlorogenic acid, neochlorogenic acid, caffeic acid, ellagic acid and dicaffeoylquinic acid. Also, condensed-type polymer polyphenols such as proanthocyanin, and hydrolyzed-type polymer polyphenols such as gallotannin and ellagitannin are encompassed in the polyphenols of the present invention. A preferred polyphenol is at least one kind selected from the group consisting of flavonoids, gallic acid, chlorogenic acid, cryptochlorogenic acid, neochlorogenic acid, caffeic acid and dicaffeoylquinic acid, from the viewpoint of exhibiting the desired effects of the present invention. The dicaffeoylquinic acid is especially preferably 3,5-dicaffeoylquinic acid. In addition, the above-mentioned flavonoid is preferably at least one kind selected from the group consisting of flavonols, flavanones, chalcones and flavanols. The above-mentioned flavonols include kaemferol, myricetin, quercetin and the like, preferably myricetin and/or quercetin. The above-mentioned flavanone is preferably isoxanthohumol. The above-mentioned chalcone is preferably xanthohumol B and/or xanthohumol D. The above-mentioned flavanol includes catechins, preferably epigallocatechin gallate. In addition, various isomers such as optical isomers, keto-enol tautomers and geometric isomers can be also used in the present invention, as long as these have enhancing action for growth factor production, and may be isolated into each isomer or may be a mixture of isomers. The polyphenol used in the present invention can be prepared by a known method. For instance, the polyphenol can be isolated and purified from fruit, seed, seed coat, flower, leaf, stem, root, or rhizome of a plant (for instance, *Artemisia L., Angelica keiskei* koidz., bamboo, plum, rice, soybean, *Chrysanthemum, Chrysanthemum coronarium, Humulus lupulus,* mulukhiya, *Curcuma zedoaeia Roscoe, Curcuma*, and the like). Also, commercially available polyphenols can be used. In addition, extracts of a plant containing a polyphenol can be directly used.

The derivative of the polyphenol in the present invention is not particularly limited. For instance, there are exemplified a carboxylic acid ester, a glycoside, a sulfated product and the like of a polyphenol. Any of these derivatives can be used in the present invention as long as they have enhancing action for growth factor production. It is preferable that the derivative is the carboxylic acid ester of a polyphenol and/or the glycoside of a polyphenol. The above-mentioned carboxylic acid ester of a polyphenol is exemplified by, for instance, an ester with a carboxylic acid having an aliphatic group, an aromatic group or an aromatic-aliphatic group. The carboxylic acid may be those having unsaturated hydrocarbons, or those of which hydrogen atom is substituted with a halogen atom or a functional group such as hydroxyl group, amino group, oxo group or the like. The carboxylic acid ester of a polyphenol may be those in which an ester is formed with all of the phenolic hydroxyl groups existing in the polyphenol, or those in which the hydroxyl group partly remains in the polyphenol. The carboxylic acid ester of the polyphenol is preferably the carboxylic acid ester of the preferred compound as the effective ingredient exemplified above as the polyphenol, more preferably caffeic acid methyl ester and/or caffeic acid ethyl ester, which are carboxylic acid esters of caffeic acid. In addition, any of carboxylic acid esters of flavonoids such as kaemferol, myricetin, quercetin, isoxanthohumol, xanthohumol B, xanthohumol D or epigallocatechin gallate are preferable.

In addition, the glycoside of a polyphenol includes, for instance, glycosides of monosaccharides such as glucose, threose, ribose, apiose, allose, ramnose, arabinopyranose, ribulose, xylose, galactose, mannose, talose, fucose, fructose, glucuronic acid and galacturonic acid, disaccharides such as neohesperidose, lutinose, agarobiose, isomaltose, sucrose, xylobiose, nigerose, maltose and lactose, polysaccharides such as agarose and fucoidan, or the like. In addition, the glycoside includes compounds in which C-C bonding is formed at a carbon other than the reducing end of the saccharide, as well as compounds in which O-, N-, S- or C-glycoside bonding is formed between the polyphenol and the saccharide. The glycoside of the polyphenol is preferably isoorientin, which is a glycoside of luteolin, a kind of flavone. Also, the glycoside of the polyphenol is preferably a glycoside of the compounds suitable as the effective ingredient exemplified above as the polyphenol. In addition, any of glycosides of flavonoids such as kaemferol, myricetin, quercetin, isoxanthohumol, xanthohumol B, xanthohumol D or epigallocatechin gallate are preferable.

Further, for instance, a compound which has such a function as a so-called prodrug, which undergoes hydrolysis reaction in the living body, thereby exhibiting enhancing action for growth factor production, is encompassed in the derivative of the polyphenol.

The derivative of the polyphenol described above can be appropriately prepared by a known method.

The salt of the polyphenol or derivative thereof according to the present invention is exemplified by, for instance, an alkali metal salt, an alkaline earth metal salt, a salt with an organic base, and the like. For instance, the salts include salts with sodium, potassium, calcium, magnesium, ammonium, diethanolamine, ethylenediamine, and the like. These salts can be obtained, for instance, by converting a phenolic hydroxyl group or the like of the polyphenol into a salt by a known method. The salt is preferably a pharmacologically acceptable salt.

Also, as the effective ingredient of the present invention, there can be suitably used the above-mentioned composition, namely a composition obtained by subjecting at least one kind selected from the group consisting of polyphenols, derivatives thereof, and salts thereof to:
(A) a mixing treatment with at least one kind selected from the group consisting of a metal, a metal salt and a metal ion, or
(B) an oxidation treatment.
By subjecting to the treatment (A) or (B) mentioned above, the enhancing action for growth factor production of the above-mentioned polyphenols or the like is enhanced. Therefore, the composition is more suitable as the effective ingredient as compared to the above-mentioned polyphenols or the like. The polyphenols or the like includes those preferred compounds as the effective ingredients exemplified as the polyphenols or the like, derivatives of the compounds, and salts thereof. Among them, chlorogenic acid and/or caffeic acid, derivatives thereof and salts thereof are preferable. Also, the derivative is preferably a carboxylic acid ester and/or a glycoside of chlorogenic acid and/or caffeic acid. The composition itself is also encompassed in the present invention.

The metal used in the present invention is not particularly limited, and a preferred metal includes at least one metal element selected from the group consisting of iron, manganese, magnesium, copper, zinc, silver, gold, aluminum, calcium, nickel and cobalt. The metal salt is preferably a salt containing the above-mentioned metal, and the metal salt includes, for instance, sulfates, acetates, nitrates, phosphates, carbonates, chlorides and the like of the metals. Also, a complex salt containing a complex ion can be used. The metal ion refers to a metal ion which is formed by dissolving these metals and/or metal salts in, for instance, an aqueous solution, and it is preferred to use at least one kind selected from the group consisting of Fe²⁺, Fe³⁺, Mn²⁺, Mn³⁺, Mg²⁺, Cu⁺, Cu²⁺, Cu³⁺, Zn²⁺, Ag⁺, Ag²⁺, Au⁺, Au³⁺, Al³⁺, Ca²⁺, Ni²⁺, Co²⁺ and Co³⁺.

The mixing treatment (A) with at least one kind selected from the group consisting of a metal, a metal salt and a metal ion (hereinafter referred to as "the metal and the like" in some cases) can be carried out by contacting the above-mentioned polyphenol and the like with the metal and the like. For instance, in an appropriate solvent, a polyphenol and a metal salt is mixed so as to have a molar ratio (polyphenol/metal salt) of preferably from 0.01 to 10000, more preferably from 1 to 100, and the mixture is allowed to stand preferably at 0° to 100°C, more preferably at room temperature (25°C), for preferably from 5 minutes to 10 days, more preferably from 30 minutes to 1 day. As the solvent, there can be used, for instance, a hydrophilic or lipophilic solvent such as water, chloroform, an alcohol such as ethanol, methanol or isopropyl alcohol, a ketone such as acetone or methyl ethyl ketone, methyl acetate or ethyl acetate, alone or as a mixed solution, and water is preferably used. In the mixing treatment, it is preferable that both the polyphenol and the like and the metal and the like which are to be treated are dissolved in the solvent. Also, in the step of extracting a polyphenol from a plant, the above-mentioned composition can also be obtained by carrying out the above-mentioned mixing treatment. For instance, the above-mentioned metal and the like are previously added to the extracting solvent, and the mixture after the extraction is allowed to stand for, for instance, several minutes to several hours, whereby the above-mentioned composition can be obtained.

Although the structure of the composition obtained by the mixing treatment is unknown, it is deduced that, for instance, the composition is formed, for instance, as a complex by some sort of bonding between the polyphenol and the like and the metal and the like, the composition is in simply mixed state without bonding, or the like.

The above-mentioned (B) oxidation treatment of the polyphenol and the like is carried out, for instance, by subjecting a polyphenol to the steps of oxidation by contact with oxygen, oxidation with an oxidizing agent, oxidation with an oxidizing enzyme, electric oxidation, or the like. Any of these steps can be carried out according to known methods. For instance, when the oxidation is carried out by contact with oxygen, the process can be carried out by feeding an air through a pump to the polyphenol and the like dissolved in an aqueous solution. The oxidation with an oxidizing agent can be carried out by suitably using as the oxidizing agent hydrogen peroxide, ozone, silver, copper, iron, nickel, manganese, cobalt, chromium or the like. The oxidation with an oxidizing enzyme can be carried out by suitably using as the oxidizing enzyme tyrosinase, peroxidase or the like. The oxidation with a microorganism containing the oxidizing enzyme exemplified above is encompassed in the oxidation with an oxidizing enzyme. When oxidation is electrically carried out, the oxidation is carried out by cathode oxidation reaction using as an electrode platinum, zinc oxide, carbon, graphite, platinum oxide or the like.

Although the structure of the composition obtained by the oxidation treatment is unknown, it is deduced that the polyphenol and the like are simply oxidized or exist as a polymer by polymerization.

The composition used as the effective ingredient is not particularly limited, as long as the composition is obtained through the treatment of (A) or (B) mentioned above. Therefore, there can be used those obtained by a treatment method including a step of treatment (A) or (B) mentioned above [namely, treatment method including steps other than the step of treatment (A) or (B) mentioned above]. In addition, the above-mentioned polyphenol and the like after the above-mentioned treatment may be directly used as a composition. On the other hand, the composition may be used after subjecting it to purification as desired in accordance with a known purification step, as long as the desired effects are obtained.

The effective ingredient according to the present invention can be used in combination with known substances having enhancing action for growth factor production. Further, a composition obtained by mixing treatment of the polyphenol and the like with the metal and the like, and a composition obtained by the oxidation treatment of the polyphenol and the like can be used in combination.

Any of the effective ingredients according to the present invention, as described above, have enhancing action for growth factor production. For instance, the exhibition of the action can be evaluated by the methods described in Examples 1 and 17 described below.

In the effective ingredient of the present invention, toxicity is not particularly recognized as described below, and there is no concern for generation of adverse actions, so that the enhancement of the growth factor production can be carried out safely and appropriately. Therefore, the therapeutic agent, the prophylactic agent, the food, the beverage or the feed of the present invention, each comprising the effective ingredient, is effective for treatment or prevention of a disease requiring the enhancement of growth factor production.

The growth factor in the present invention is not particularly limited, as long as the growth factor has activity for accelerating the cell growth. The growth factor is exemplified by hepatocyte growth factor (HGF), nerve growth factor (NGF), neurotrophic factor, epidermal growth factor, milk-derived growth factor, fibroblast growth factor, brain-derived fibroblast growth factor, acidic fibroblast growth factor, platelet-derived growth factor, platelet basic protein, connective tissue-activating peptide, insulin-like growth factor, colony-stimulating factor, erythropoietin, thrombopoietin, T cell growth factor, interleukins (for instance, interleukins 2, 3, 4, 5, 7, 9, 11 and 15), B cell growth factor, cartilage-derived factor, cartilage-derived growth factor, bone-derived growth factor, skeletal growth factor, epithelial cell growth factor, epithelial cell-derived growth factor, oculus-derived growth factor, testis-derived growth factor, Sertoli's cell-derived growth factor, mammotropic factor, spinal cord-derived growth factor, macrophage-derived growth factor, mesodermal growth factor, transforming growth factor-α, transforming growth factor-β, heparin-binding EGF-like growth factor, amphyllegrin, SDGF, betacellulin, epiregulin, neuregulin 1, 2 and 3, vascular endotherial growth factor, neurotrophin, BDNF, NT-3, NT-4, NT-5, NT-6, NT-7, glial cell line-derived neurotrophic factor, stem cell factor, midkine, pleiotrophin, ephrin, angiopoietin, activin, tumor necrosis factor, interferons, and the like. According to the present invention, the enhancement for production of nerve growth factor (NGF) or hepatocyte growth factor (HGF) can be especially suitably carried out.

HGF is a factor for hepatocyte regeneration, and is further a factor for facilitating motility of cells, as well as a tumor cytotoxic factor. HGF accelerates growth of many of epithelial cells, such as changioepithelial cells, renal tubule epithelial cells, and gastric mucosa cells, as well as hepatocytes. In addition, HGF exhibits remarkably a wide variety of physiological activity, for instance, HGF induces morphological formations as seen in facilitation of motility of epithelial cells, vascularization or luminal formation of epithelial cells. Therefore, by enhancing the production of HGF, hepatic disorders such as hepatitis, severe hepatitis, fulminant hepatitis, cirrhosis, and cholestasia in the liver, renal disorders caused by drugs and the like, gastrointestinal disorders, vascular disorders, chronic nephritis, pneumonia, wound, diabetes, cancer, and the like can be treated or prevented.

On the other hand, NGF is an endogenous growth factor for maintaining viability and functions of nerve cells, elongating nerve cells in accordance with a concentration gradient of NGF, or the like. By enhancing the production of NGF, the treatment or prevention of senile dementia such as Alzheimer's disease, peripheral nerve disorder, cerebrovascular disorder, cerebral tumor, cerebral apicitis, nerve degenerative disease caused by head injury, diseases requiring recovery and regeneration of nerve functions, caused by intoxication with an anesthetic, and the like can be carried out. In addition, it is useful in the treatment or prevention of amyotrophic lateral sclerosis, drug-induced peripheral nerve disorder, diabetic peripheral nerve disorder, Parkinson's disease, sensory nerve disorder, retinitis pigmentosa, macular dystrophy, and the like.

The disease requiring the enhancement of growth factor production in the present invention is not particularly limited, as long as the disease can be treated or prevented by enhancing growth factor production with the therapeutic agent, the prophylactic agent or the like, comprising the above-mentioned effective ingredient. It is especially effective for various diseases exemplified above which can be treated or prevented by enhancing HGF or NGF production.

The therapeutic agent or prophylactic agent of the present invention for a disease requiring enhancement of growth factor production includes those formed into a preparation by combining the above-mentioned effective ingredient with a known pharmaceutical vehicle. In this embodiment, as the salt which is the effective ingredient, the pharmacologically acceptable salts can be used.

The therapeutic agent or prophylactic agent of the present invention is usually prepared by formulating the above-mentioned effective ingredient with a pharmacologically acceptable liquid or solid vehicle, and optionally adding thereto a solvent, a dispersant, an emulsifier, a buffer, a stabilizer, an excipient, a binder, a disintegrant, a lubricant, or the like, thereby being made into a solid agent such as a tablet, a granule, a powder, a fine powder, and a capsule, or a liquid agent such as a common liquid agent, a suspension agent or an emulsion agent. In addition, there can be also prepared a dry product which can be made liquid by adding an appropriate vehicle before use, and an external preparation.

The pharmaceutical vehicle can be selected depending upon the above-mentioned administration form and preparation form of the therapeutic agent or prophylactic agent. In the case of an orally administered preparation comprising a solid composition, a tablet, a pill, a capsule, a powder, a fine powder, a granule or the like can be formed by utilizing, for instance, starch, lactose, saccharose, mannitol, carboxymethyl cellulose, cornstarch, an inorganic salt or the like. In addition, during the preparation of the orally administered preparation, a binder, a disintegrant, a surfactant, a lubricant, a fluidity accelerator, a flavor, a colorant, a perfume, and the like can be further formulated. In the case of forming into a tablet or pill, for instance, the tablet or pill may be covered with a sugar-coating made of sucrose, gelatin or hydroxypropyl cellulose, or with a film made of a substance soluble in the stomach or intestine as occasion demands. In the case of an orally administered preparation comprising a liquid composition, the preparation can be prepared in the form of a pharmaceutically acceptable emulsion, solution, suspension, syrup, or the like, using purified water, ethanol, or the like, for instance, as a vehicle. Furthermore, an auxiliary agent such as a wetting agent or a suspending agent, a sweetener, a flavor, an antiseptic, or the like may be added as desired.

On the other hand, a non-orally administered preparation is prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, by a conventional method, and adding a microbicide, a stabilizer, an osmotic regulator, a soothing agent, or the like as necessary. It is also possible to produce a solid composition which is dissolved in sterile water or a sterile solvent for injection before use.

The external preparation includes solid, semi-solid or liquid preparations for percutaneous administration or transmucosal (oral or intranasal) administration. The external preparation also includes suppositories and the like. For instance, the external preparation may be prepared as liquid preparations including emulsions, suspensions such as lotions, external tinctures, and liquids for transmucosal administration; ointments including oily ointments and hydrophilic ointments; medical adhesives for percutaneous administration or transmucosal administration such as films, tapes and poultices; and the like.

Each of the above-mentioned various preparations can be appropriately produced by conventional methods by utilizing known pharmaceutical vehicles and the like. The content of the effective ingredient in the preparation is not particularly limited, as long as the content is preferably the amount so that the effective ingredient can be administered within the dose described below in consideration of administration form, administration method and the like of the preparation.

The therapeutic agent or prophylactic agent of the present invention is administered via an administration route appropriate for each of the preparation form. The administration route is not limited to specific one. The agent can be administered internally or externally (or topically) or by injection. The injection can be administered, for instance, intravenously, intramuscularly, subcutaneously, intracutaneously, or the like. In the use of external preparations, for instance, a suppository may be administered by a method of administration suitable therefor.

The dose for the therapeutic agent or prophylactic agent of the present invention is changeable and properly set depending upon its preparation form, administration method, purpose of use, age, body weight, symptom or the like of the patient to which the therapeutic agent or prophylactic agent is applied, or the like. Generally, the dose for the above-mentioned effective ingredient contained in the preparation is preferably from 0.1 µg to 200 mg/kg weight per day for adult. As a matter of course, the dose varies depending upon various conditions, so that an amount smaller than the dose mentioned above may be sufficient, or an amount exceeding the dose range may be required. The agent may be administered in a single dose or in several divided portions during the day within the desired dose range. Also, the agent of the present invention can be directly orally administered, or the agent can be added to any foodstuffs to take it on a daily basis.

In addition, in another embodiment of the present invention, there can provided an enhancer for growth factor production comprising the above-mentioned effective ingredient according to the present invention. The enhancer may be the above-mentioned effective ingredient itself, or a composition comprising the above-mentioned effective ingredient. In this embodiment, as the salt which is the effective ingredient, the pharmacologically acceptable salts can be preferably used. The enhancer for growth factor production can be produced by formulating the above-mentioned effective ingredient with other ingredients which can be used for the same application as the effective ingredient, and forming into a form of reagent usually used according to the above-mentioned process for producing the therapeutic agent or prophylactic agent. The content of the above-mentioned effective ingredient in the enhancer is not particularly limited, as long as the content is in an amount so that the desired effects of the present invention can be exhibited in consideration of administration route, administration purpose or the like of the enhancer. Also, the amount of the enhancer used is not particularly limited, as long as the desired effects of the present invention can be exhibited. Especially in the case where the enhancer is administered to a living body, the enhancer is preferably used in an amount so that the effective ingredient can be administered within the dose range of the effective ingredient in the above-mentioned therapeutic agent or prophylactic agent. The enhancer for growth factor production is useful for enhancement of growth factor production, especially useful for enhancement of the production of growth factor in a disease requiring enhancement for HGF or NGF production. In addition, the enhancer is also useful for screening of drugs for growth factor-associated diseases. Also, the enhancer can be used for a method for activating epithelial cells, and this method is useful for biochemical studies relating to epithelial cell mechanism, and screening drugs for hepatic disorders, renal disorders, gastrointestinal disorders, and vascular disorders. In addition, the enhancer is useful for functional studies of growth factor.

Further, in a still another embodiment of the present invention, there can provided a method for enhancing growth factor production, comprising administering the above-mentioned effective ingredient according to the present invention to an animal. In this embodiment, as the salt which is the effective ingredient, the pharmacologically acceptable salts can be preferably used. This method can be carried out by administering the above-mentioned effective ingredient, preferably as the above-mentioned enhancer for growth factor production, to an animal that is predicted to require or requires enhancement of growth factor production, so that the growth factor production is enhanced by the administration. The administration method, dose, or the like of the effective ingredient may be similar to that of the above-mentioned enhancer for growth factor production. In the method for enhancing growth factor production, the therapeutic agent or prophylactic agent, or the food, beverage or feed described below of the present invention can be used. In addition, the term "animal" includes a mammal such as human, dogs, cats, *Bos, Porcus, Equus,* and the like, among which the method is preferably used for human. The method for enhancing growth factor production is useful for, for instance, the enhancement of growth factor production in the treatment or prevention of a disease requiring enhancement for growth factor production. In addition, the method is also useful for screening of drugs for growth factor-associated diseases. Also, the method is useful for functional studies of growth factor.

In addition, the present invention provides a food, beverage or feed for enhancing growth factor production, comprising the above-mentioned effective ingredient. In this embodiment of the present invention, as the salt which is the effective ingredient, a pharmacologically acceptable salt or a salt having the same level of safety as the pharmacologically acceptable salt can be suitably used. Since the food, beverage or feed has enhancing action for growth factor production, the food, beverage or feed is very useful in amelioration or prevention of symptoms for a disease requiring enhancement for growth factor production.

Here, in this embodiment of the present invention, the term "comprise or comprising" includes the meanings of containing, adding and diluting. The term "containing" refers to an embodiment of containing the effective ingredient used in the present invention in the food, beverage or feed; the term "adding" refers to an embodiment of adding the effective ingredient used in the present invention to a raw material for the food, beverage or feed; and the term "diluting" refers to an embodiment of adding a raw material for the food, beverage or feed to the effective ingredient used in the present invention.

The method for preparing the food, beverage or feed of the present invention is not particularly limited. For instance, formulation, cooking, processing, and the like can be carried out in accordance with those generally employed for foods, beverages or feeds, and the food, beverage or feed can be prepared by the general methods for preparing a food, beverage or feed, as long as the resulting food, beverage or feed may contain the above-mentioned effective ingredient according to the present invention, wherein the effective ingredient has enhancing action for growth factor production.

The food or beverage of the present invention is not particularly limited. The food or beverage includes, for instance, processed agricultural and forest products, processed stock raising products, processed marine products and the like, including processed grain products such as processed wheat products, processed starch products, processed premix products, noodles, macaronis, bread, bean jam, buckwheat noodles, wheat-gluten bread, rice noodle, *fen-tiao*, and packed rice cake; processed fat and oil products such as plastic fat and oil, tempura oil, salad oil, mayonnaise, and dressing; processed soybean products such as tofu products, soybean paste, and fermented soybeans; processed meat products such as ham, bacon, pressed ham, and sausage; marine products such as frozen ground fish, boiled fish paste, tubular roll of boiled fish paste, cake of ground fish, deep-fried patty of fish paste, fish ball, sinew, fish meat ham and sausage, dried bonito, products of processed fish egg, marine cans, and preserved food boiled down in soy sauce (*tsukudani*); milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks, and rice snacks; alcohol beverages such as *sake,* Chinese liquor, wine, whisky, Japanese distilled liquor (*shochu*), vodka, brandy, gin, ram, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, binned or pouched foods such as rice topped cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste (*miso*) soup, precooked foods, precooked beverages, and precooked soup; frozen foods such as *sukiyaki*, pot-steamed hotchpotch, split and grilled eel, hamburger steak, *shao-mai*, dumpling stuffed with minced pork, various sticks, and fruit cocktails; solid foods; liquid foods (soups); spices; and the like, wherein the food or beverage comprises the above-mentioned effective ingredient according to the present invention.

The above-mentioned effective ingredient is singly or in plurality contained, added and/or diluted in the food or beverage. The food or beverage of the present invention does not have any particular limitation on its shape, as long as the effective ingredient is contained in an amount necessary for the food or beverage to exhibit enhancing action for growth factor production. Such shapes also include orally taken shapes such as tablets, granules and capsules.

The content of the above-mentioned effective ingredient in the food or beverage of the present invention is not particularly limited, and the content can be appropriately selected from the viewpoints of sensory ability and exhibition of activity. The content of the effective ingredient is, for instance, preferably 0.0001 parts by weight or more, more preferably from 0.001 to 10 parts by weight, per 100 parts by weight of the food, or for instance, preferably 0.0001 parts by weight or more, more preferably from 0.001 to 10 parts by weight, per 100 parts by weight of the beverage. Also, the food or beverage of the present invention may be taken such that the effective ingredient contained therein is taken in an amount of preferably from 0.01 to 100 mg/kg weight per day for adult.

In addition, the present invention provides a feed for an organism having enhancing action for growth factor production, in which the above-mentioned effective ingredient is contained, added and/or diluted. In another embodiment, the present invention also provides a method of feeding an organism, characterized by administering the above-mentioned effective ingredient to the organism. In still another embodiment, the present invention provides an organism feeding agent characterized in that the organism feeding agent comprises the above-mentioned effective ingredient.

In these inventions, the organism includes, for instance, culturing or breeding animals, pet animals, and the like. The culturing or breeding animal is exemplified by cattle, experimental animals, poultry, pisces, crustaceae or shellfish. The feed is exemplified by a feed for sustenance of and/or improvement in physical conditions. The organism feeding agent includes immersion agents, feed additives, and beverage additives.

According to these inventions, the same effects can be expected to be exhibited as those of the above-mentioned therapeutic agent or prophylactic agent of the present invention, on the basis of the enhancing action for growth factor production of the above-mentioned effective ingredient used in the present invention, in the organism exemplified above for applying these. In other words, according to these inventions, there can be expected exhibition of therapeutic or prophylactic effect for a disease requiring enhancing action for growth factor production in the organism.

The above-mentioned effective ingredient used in the present invention is usually administered in an amount of preferably from 0.01 to 2000 mg per 1 kg of the body weight of the subject organism per day. The administration can be made by adding and mixing the effective ingredient in a raw material for an artificially formulated feed, or mixing the effective ingredient with a powder raw material for an artificially formulated feed, and thereafter further adding and mixing the resulting mixture with other raw materials, wherein the artificially formulated feed is applied to a subject organism. In addition, the content of the above-mentioned effective ingredient in the feed is not particularly limited. The content of the effective ingredient can be appropriately set in accordance with its purposes, and an appropriate proportion in the feed is from 0.001 to 15% by weight.

The artificially formulated feed includes feeds using as raw materials animal-derived raw materials such as fish meal, casein, and squid meal; plant-derived raw materials such as soybean grounds, flour, and starch; microorganism raw materials such as yeasts for feed; animal fats and oils such as cod-liver oil and squid-liver oil; vegetable fats and oils such as soybean oil and rapeseed oil; and other raw materials such as vitamins, minerals, amino acids, and antioxidants; and the like. In addition, feeds for fish such as fish minced meat are also included.

The method for preparing the feed of the present invention is not particularly limited. In addition, the formulation may be in accordance with those of general feeds, as long as the above-mentioned effective ingredient according to the present invention having enhancing action for growth factor production is contained in the feed produced.

Also, the above-mentioned effective ingredient having enhancing activity for growth factor production according to the present invention can be administered by directly adding the above-mentioned effective ingredient to water, seawater, or the like in a pool, a water tank, a water reservoir, or a feeding range, and immersing a subject organism into the resulting solution. The immersion method is especially effective when the amount of intake of the feed of the subject organism is lowered. The concentration of the effective ingredient according to the present invention having enhancing action for growth factor production in water or seawater is not particularly limited. The concentration of the effective ingredient may be adjusted in accordance with its purposes. It is appropriate that the concentration is preferably from 0.00001 to 1% by weight.

Also, a beverage comprising the above-mentioned effective ingredient according the present invention, having enhancing action for growth factor production may be given to a subject organism as a feeding drink. The concentration of the effective ingredient used in the present invention having enhancing action for growth factor production in the beverage is not particularly limited. The concentration of the effective ingredient may be adjusted in accordance with its purposes. It is appropriate that the concentration is preferably from 0.0001 to 1% by weight. The organism feeding agent, for instance, an immersion agent, a feed additive, or a beverage additive comprising the above-mentioned effective ingredient according to the present invention having enhancing action for growth factor production may be prepared by known formulation and preparation method. The content of the effective ingredient in the organism feeding agent is not particularly limited, so long as the desired effects of the present invention can be obtained.

The organism to which the present invention can be applied is not limited. The culturing or breeding animals include cattle such as *Equus, Bos, Porcus, Ovis, Capra, Camelus,* and *Lama*; experimental animals such as mice, rats, guinea pigs, and rabbits; poultry such as *Chrysolophus,* ducks, *Meleagris*, and *Struthioniformes;* pisces such as *Pagrus, Oplegnathidae, Paralichthys*, plaice, *Seriola*, young *Seriola*, amberjack, *Thunna, Caranx delicatissimus, Plecoglossus, Salmo•Oncorhynchus, Fugu, Anguilla, Misguirus,* and *Parasilurus*; Crustaceae such as *Penaidae,* black tiger shrimp, *Penaeus roentalis,* and *Portulus trituberculatus*; and shellfish such as abalones (*awabi*), turban shells, scallops, and oysters; and the pet animals includes dogs, cats, and the like, so that the feed can be widely applied to animals on land and in water.

By allowing a subject organism to take the feed comprising the above-mentioned effective ingredient used in the present invention having enhancing action for growth factor production, or immersing a subject organism into a solution containing the above-mentioned effective ingredient used in the present invention having enhancing action for growth factor production, the physical conditions of the cattle, experimental animals, poultry, pisces, Caustacea, shellfish, pet animals or the like can be well sustained and ameliorated.

Further, as a still another embodiment, the present invention provides use of the above-mentioned effective ingredient according to the present invention in the preparation of a therapeutic agent or prophylactic agent for a disease requiring enhancement of growth factor production, an enhancer for growth factor production, or a food, beverage or feed for enhancing growth factor production. The use embodiments include use embodiments of the above-mentioned effective ingredient in the preparation of the therapeutic agent or prophylactic agent, the enhancer for growth factor production, or the food, beverage or feed for enhancing growth factor production of the present invention mentioned above. For instance, as the use of the above-mentioned effective ingredient in the preparation of a therapeutic agent or prophylactic agent for a disease requiring enhancement of growth factor production, or an enhancer for growth factor production, there are exemplified the use in the preparation of a solid agent such as a tablet, a granule, a powder, a fine powder, and a capsule, a liquid agent such as a common liquid agent, a suspension agent, or an emulsion agent, or a dry product which can be liquefied by adding an appropriate vehicle before use.

The above-mentioned effective ingredient used in the present invention shows no toxicity, even when its effective dose for its action and exhibition is administered. For instance, there are no cases of death when any of kaemferol, myricetin, quercetin, isoxanthohumol, xanthohumol B, xanthohumol D, epigallocatechin gallate, gallic acid, chlorogenic acid, cryptochlorogenic acid, neochlorogenic acid, caffeic acid, dicaffeoylquinic acid, isoorientin, methyl ester caffeic acid, ethyl ester caffeic acid, or these optical activated isomers or salts thereof is orally administered to a mouse in a single dose of 1 g/1 kg.

### EXAMPLES

The present invention will be more concretely described below by means of Examples, without intending to limit the present invention thereto. Here, "%" in Examples means "% by weight" unless otherwise specified.

### Example 1 Enhancing Activity for HGF Production of Myricetin and Quercetin

MRC-5 cells (CCL 171: manufactured by DAINIPPON PHARMACEUTICAL CO., LTD., code. 02-021) were suspended in a DME medium containing 10% fetal bovine serum so as to have a concentration of 5 × 10⁴ cells/cm². The suspension was put in a 48-well cell culture plate, and the cells were cultured at 37°C in the presence of 5% CO₂ gas for 24 hours. After culturing, the medium was exchanged. Thereafter, the sample was added, and the cells were cultured for another 24 hours. Subsequently, the medium was collected, and the amount of HGF in the medium was assayed using Quantikine Human Hepatocyte Growth Factor (HGF) ELISA Kit (manufactured by Funakoshi, code. RS-0641-00). As the sample, myricetin (Sample (i)) was added so as to have a final concentration of 0, 1, 10 or 100 µM, and quercetin (Sample (ii)) was added so as to have a final concentration of 0, 10 or 100 µM. Here, the addition of the sample was carried out by previously preparing aqueous solutions of the samples at various concentrations, and adding a given amount of each solution to the medium so as to have the above-mentioned final concentration. As the control, that with addition of a given amount of distilled water alone (final concentration of the sample: 0 µM) was used. The amount of HGF production was expressed as the relative value (%) when the HGF concentration in the cell culture medium of the control was defined as 100%. The results are shown in Table 1. All experiments were carried out twice, and an average value was taken. As shown in Table 1, the samples (i) and (ii) enhanced HGF production. Further, myricetin, which has a larger number of hydroxyl groups in the B-ring of the flavonol, had a higher enhancing activity for HGF production.

From this, it was demonstrated that the flavonols and the derivatives thereof had an enhancing activity for HGF production.

**Table 1**

| Sample | Concentration (µM) | Amount of HGF Production (%) |
|---|---|---|
| Sample (i) | 0 | 100 |
| | 1 | 187 |
| | 10 | 543 |
| | 100 | 454 |
| Sample (ii) | 0 | 100 |
| | 10 | 139 |
| | 100 | 304 |
| (Here, the amount of HGF production in the control was 4.80 ng/ml.) | | |

### Example 2 Enhancing Activity for HGF Production of Epigallocatechin Gallate

The enhancing activity for HGF production of epigallocatechin gallate was assayed in the same manner as in Example 1. Here, epigallocatechin gallate was added so as to have a final concentration of 0, 1 or 10 µM. The results are shown in Table 2. As shown in Table 2, epigallocatechin gallate enhanced HGF production.

**Table 2**

| Concentration (µM) | Amount of HGF Production (%) |
|---|---|
| 0 | 100 |
| 1 | 175 |
| 10 | 160 |
| (Here, the amount of HGF production in the control was 6.78 ng/ml.) | |

### Example 3 Enhancing Activity for HGF Production of Gallic Acid

The enhancing activity for HGF production of gallic acid was assayed in the same manner as in Example 1. Here, gallic acid was added so as to have a final concentration of 0, 1, 10 or 100 µM. The results are shown in Table 3. As shown in Table 3, gallic acid enhanced HGF production.

**Table 3**

| Concentration (µM) | Amount of HGF Production (%) |
|---|---|
| 0 | 100 |
| 1 | 112 |
| 10 | 217 |
| 100 | 576 |
| (Here, the amount of HGF production in the control was 6.78 ng/ml.) | |

### Example 4 Enhancing Activity for HGF Production of Chlorogenic Acid

The enhancing activity for HGF production of chlorogenic acid was assayed in the same manner as in Example 1. Chlorogenic acid (manufactured by Tokyo Kasei) was added so as to have a final concentration of 0, 1, 10, 100, 500 or 1000 µM. The results are shown in Table 4. As shown in Table 4, chlorogenic acid enhanced HGF production.

**Table 4**

| Concentration (µM) | Amount of HGF Production (%) |
|---|---|
| 0 | 100 |
| 1 | 105 |
| 10 | 122 |
| 100 | 302 |
| 500 | 479 |
| 1000 | 624 |
| (Here, the amount of HGF production in the control was 5.16 ng/ml.) | |

### Example 5 Enhancing Activity for HGF Production of Caffeic Acid

The enhancing activity for HGF production of caffeic acid was assayed in the same manner as in Example 1. Here, caffeic acid (manufactured by Sigma) was added so as to have a final concentration of 0, 10 or 100 µM. The results are shown in Table 5. As shown in Table 5, caffeic acid enhanced HGF production.

**Table 5**

| Concentration (µM) | Amount of HGF Production (%) |
|---|---|
| 0 | 100 |
| 10 | 117 |
| 100 | 196 |
| (Here, the amount of HGF production in the control was 6.27 ng/ml.) | |

### Example 6 Preparation and Enhancing Activity for HGF Production of Dicaffeoylquinic Acid

(1) Forty grams of a dried product of *Altemisia princeps pampan* (manufactured by Sakamoto Kanpodo) was extracted three times with 500 mL of 50% acetone, and thereafter the extract was concentrated under reduced pressure to a volume of about 15 mL. Thirty-five milliliters of a 10% aqueous citric acid was added to the concentrate, and the resulting mixture was extracted three times with 100 mL of ethyl acetate. Thereafter, the organic layer was dried over magnesium sulfate, and concentrated under reduced pressure. The concentrate was subjected to silica chromatography using a developing solvent of chloroform:methanol:acetic acid = 2:1:1 (volume ratio), and the eluate was collected 8 mL each. Fractions 7 to 13 obtained were combined, and concentrated under reduced pressure. Thereafter, the concentrate was developed on a silica gel plate using a developing solvent of chloroform:methanol:acetic acid = 1:1:0.05, to recover 225 mg of a substance located near the Rf value of 0.5, showing absorption at UV 254 nm. The structure of this substance was analyzed by ¹H-NMR. As a result, the substance was found to be dicaffeoylquinic acid (3,5-dicaffeoylquinic acid).
(2) The enhancing activity for HGF production of dicaffeoylquinic acid prepared in item (1) of Example 6 was assayed in the same manner as in Example 1. Here, dicaffeoylquinic acid was added so as to have a final concentration of 0, 1, 10 or 100 µM. The results are shown in Table 6. As shown in Table 6, dicaffeoylquinic acid enhanced HGF production.

**Table 6**

| Concentration (µM) | Amount of HGF Production (%) |
|---|---|
| 0 | 100 |
| 1 | 142 |
| 10 | 206 |
| 100 | 290 |
| (Here, the amount of HGF production in the control was 6.27 ng/ml.) | |

### Example 7 Preparation and Enhancing Activity for HGF Production of Dicaffeoylquinic Acid

A raw sample of about 900 g of *Chrysanthemum coronarium* was lyophilized, and the lyophilized sample was grinded together with 2 L of 80% ethanol with a mixer. The resulting mixture was filtered using a gauze, and the resulting extract was concentrated to a volume of 200 mL, to give a crude extract of *Chrysanthemum coronarium*. A 100-ml portion of the resulting crude extract was applied to a 200 mL XAD resin (manufactured by Organo), and the elution was carried out with 500 mL each of 0%, 30%, 40%, 60% or 100% methanol. Each fraction was concentrated to a volume of 50 mL, and the enhancing activity for HGF production of each fraction was confirmed in the same manner as in Example 1. As a result, as shown in Table 7, the 60% methanol-eluted fraction had an enhancing activity for HGF production. The 60% methanol-eluted fraction was analyzed by ¹H-NMR. As a result, it was found that this fraction contained 3,5-dicaffeoylquinic acid in a high concentration. Here, the fraction was added so as to have a final concentration of 0, 0.1 or 1%.

**Table 7**

| 60% Methanol Eluted-Fraction of *Chrysanthemum Coronarium* Crude Extract (%) | Amount of HGF Production (%) |
|---|---|
| 0 | 100 |
| 0.1 | 220 |
| 1 | 349 |
| (Here, the amount of HGF production in the control was 8.22 ng/ml.) | |

### Example 8 Enhancing Activity for HGF Production of Isoorientin

About 5 g of thinly cut bamboo blades were extracted with 70 mL of 50% acetone for 15 minutes or more with stirring. The resulting extract was filtered with suction, and the residue was extracted again with the same volume of the solvent. A 1 ml portion of 100 mL of the resulting filtrate was dried under reduced pressure, and thereafter the resulting product was dissolved in 1 mL of DMSO. The resulting solution was assayed for the enhancing activity for HGF production in the same manner as in Example 1. As a result, as shown in Table 8, the enhancing activity for HGF production was confirmed. The crude extract was added so as to have a final concentration of 0, 0.1 or 1%. The crude extract was concentrated under reduced pressure, and the concentrate was 20-fold diluted with water. A portion of the dilution was applied to 10 mL XAD-2 (manufactured by Organo), and the elution was carried out with 30 mL each of 30%, 40%, 50%, 60% or 100% methanol. The enhancing activity for HGF production of each fraction was assayed in the same manner as in Example 1. As a result, the activity was confirmed in 40% methanol-eluted fraction or 30% methanol-eluted fraction. Each of the 30% methanol-eluted fraction and 40% methanol-eluted fraction was concentrated, dried and subjected to silica column chromatography using a developing solvent of chloroform:methanol:acetic acid = 3:1:0.025. The eluate was collected 10 mL each. As a result of analysis by ¹H-NMR and FAB-MS, the 19th to the 35th fractions contained isoorientin, a glycoside of luteolin which is a kind of flavones, in a high concentration, and it was confirmed that these fractions had an enhancing activity for HGF production as shown in Table 9. Here, the fraction was added so as to have a final concentration of 0, 0.1 or 1 mg/ml.
¹H-NMR
isoorientin : σ 7.57 (1H, dd, J = 8.0, 2.0 Hz), 7.55 (1H, d, J = 2.0 Hz), 7.02 (1H, d, J = 8.0 Hz), 6.81 (1H, s), 6.63 (1H, d, J = 8.0 Hz), 4.74 (1H, d, J = 10.0 Hz), 4.19 (1H, t, J = 4.5 Hz)
FAB-MASS
449 (M + H)⁺

**Table 8**

| Bamboo Blade Crude Extract (%) | Amount of HGF Production (%) |
|---|---|
| 0 | 100 |
| 0.1 | 195 |
| 1 | 463 |
| (Here, the amount of HGF production in the control was 9.09 ng/ml.) | |

**Table 9**

| Fraction Containing Isoorientin in High Concentration (mg/ml) | Amount of HGF Production (%) |
|---|---|
| 0 | 100 |
| 0.1 | 131 |
| 1 | 318 |
| (Here, the amount of HGF production in the control was 9.62 ng/ml.) | |

### Example 9 Enhancing Activity for HGF Production of Isomer of Chlorogenic Acid

About 300 g of dried plums were grinded together with 500 mL of 100% methanol with a mixer. The resulting mixture was filtered with suction, to obtain its filtrate. One milliliter of the filtrate was concentrated to dryness, and the resulting product was dissolved in 1 mL of DMSO. The enhancing activity for HGF production of this plum crude extract was assayed in the same manner as in Example 1. As a result, as shown in Table 10, the activity was confirmed. The crude extract was added so as to have a final concentration of 0, 0.01, 0.1 or 1%.

The resulting crude extract was concentrated to remove extraction solvent, and the resulting concentrate was diluted with water to a volume of 1 L. The dilution was applied to 200 mL XAD-2, and washed with 500 mL of water. Thereafter, the adsorbed fraction was eluted with 100% methanol. The resulting eluted fractions were concentrated, and thereafter the concentrate was dissolved in a small amount of water. Subsequently, the solution was subjected to a column containing Cosmosil 140 C₁₈-OPN (manufactured by nakalaitesque), and the elution was carried out with a gradient of from 0% to 25% acetonitrile/water in a total amount of 800 mL. The eluate was collected about 10 mL each. The resulting fractions were analyzed by ¹H-NMR. As a result, the 26th to the 32nd fractions and the 41st to the 48th fractions contained neochlorogenic acid (5-caffeoyl-quinic acid) and cryptochlorogenic acid (4-caffeoyl-quinic acid), respectively, which are isomers of chlorogenic acid, in a high concentration. Also, it was confirmed that both fractions had enhancing activities for HGF production as shown in Tables 11 and 12, respectively. Here, these fractions were added so as to have a final concentration of 0, 0.01 or 0.1 mg/ml.
¹H-NMR
5-caffeoyl-quinic acid : σ 7.63 (1H, d, J = 15.5 Hz), 7.19 (1H, s), 7.12 (1H, d, J = 8.0 Hz), 6.93 (1H, d, J = 8.0 Hz), 5.40 (1H, m), 4.18 (1H, m), 3.77 (1H, dd, J = 9.5, 4.5 Hz), 2.2-1.6 (5H, m)
4-caffeoyl-quinic acid : σ 7.49 (1H, d, J = 15.5 Hz), 7.04 (1H, d, J = 4.0 Hz), 6.99 (1H, dd, J = 8.0, 4.0 Hz), 6.73 (1H, d, J = 8.0 Hz), 6.27 (1H, d, J = 16 Hz), 4.65 (1H, dd, J = 8.0, 3.0 Hz), 4.08 (1H, m), 2.2-1.6 (5H, m)

**Table 10**

| Plum Crude Extract (%) | Amount of HGF Production (%) |
|---|---|
| 0 | 100 |
| 0.01 | 111 |
| 0.1 | 214 |
| 1 | 306 |
| (Here, the amount of HGF production in the control was 8.02 ng/ml.) | |

**Table 11**

| Fraction Containing Neochlorogenic Acid in High Concentration (mg/ml) | Amount of HGF Production (%) |
|---|---|
| 0 | 100 |
| 0.01 | 111 |
| 0.1 | 321 |
| (Here, the amount of HGF production in the control was 9.98 ng/ml.) | |

**Table 12**

| Fraction Containing Cryptochlorogenic Acid in High Concentration (mg/ml) | Amount of HGF Production (%) |
|---|---|
| 0 | 100 |
| 0.01 | 103 |
| 0.1 | 142 |
| (Here, the amount of HGF production in the control was 9.98 ng/ml.) | |

### Example 10 Preparation of Injections

### Injections

(1) To physiological saline was added myricetin, quercetin, kaempferol, epigallocatechin gallate, gallic acid, chlorogenic acid, neochlorogenic acid, cryptochlorogenic acid, isoxanthohumol, xanthohumol B, xanthohumol D, caffeic acid or dicaffeoylquinic acid so as to have a concentration of 1%, to prepare each injection.
(2) To physiological saline was added myricetin, quercetin, kaempferol, epigallocatechin gallate, gallic acid, chlorogenic acid, neochlorogenic acid, cryptochlorogenic acid, isoxanthohumol, xanthohumol B, xanthohumol D, caffeic acid or dicaffeoylquinic acid, and glycyrrhizic acid so as to have concentrations of 0.5% and 0.1%, respectively, to give an injection.

### Example 11 Preparation of Tablets

### Tablets

(1) A tablet containing 100 mg of myricetin, quercetin, kaempferol, epigallocatechin gallate, gallic acid, chlorogenic acid, neochlorogenic acid, cryptochlorogenic acid, isoxanthohumol, xanthohumol B, xanthohumol D, caffeic acid or dicaffeoylquinic acid, and an appropriate amount of microcrystalline cellulose was prepared, and covered with a sugar, to give each tablet.
(2) A tablet containing 0.1 mg each of myricetin, quercetin, kaempferol, epigallocatechin gallate, gallic acid, chlorogenic acid, neochlorogenic acid, cryptochlorogenic acid, isoxanthohumol, xanthohumol B, xanthohumol D, caffeic acid or dicaffeoylquinic acid, 10 mg of dipotassium glycyrrhizate and an appropriate amount of microcrystalline cellulose was prepared, and covered with a sugar, to prepare a tablet.

### Example 12 Enhancement of Enhancing Activity for HGF Production of Chlorogenic Acid by Iron

An aqueous chlorogenic acid was prepared so as to have a concentration of 100 mM. Next, a 10 mM aqueous ferric chloride (manufactured by Nakarai Chemical Ltd.) was prepared, and mixed with an equal volume of the aqueous chlorogenic acid. The resulting mixture was allowed to stand at room temperature for 60 minutes. The enhancing activity for HGF production of this iron-treated chlorogenic acid was assayed in the same manner as in Example 1. The iron-treated chlorogenic acid was added so as to have a concentration of 0, 10, 100 or 500 µM when calculated as chlorogenic acid. Incidentally, the addition of the sample was carried out by previously preparing aqueous solutions of iron-treated chlorogenic acid at various concentrations, and adding a given amount of each solution to the medium so as to have a final concentration mentioned above. In the case where the final concentration of chlorogenic acid was 0 µM, a given amount of an aqueous solution obtained by an iron treatment in the absence of chlorogenic acid was added to the medium. In addition, for the purpose of comparison, the activity of the chlorogenic acid without the iron treatment was assayed in the same manner. As described above, that with addition of a given amount of distilled water alone (final concentration of chlorogenic acid: 0 µM) was used as the control. The amount of HGF production was expressed as the relative value (%) when the HGF concentration in the cell culture medium of the control was defined as 100%. The results are shown in Table 13. The enhancing activity for HGF production of chlorogenic acid was enhanced by the iron treatment.

**Table 13**

| Concentration (µM) | | 0 | 10 | 100 | 500 |
|---|---|---|---|---|---|
| Without Iron Treatment | Amount of HGF Production (%) | 100 | 122 | 302 | 479 |
| Iron Treatment | Amount of HGF Production (%) | 77 | 399 | 508 | 635 |
| (Here, the amount of HGF production in the control was 5.16 ng/ml.) | | | | | |

### Example 13 Enhancement of Enhancing Activity for HGF Production of Chlorogenic Acid by Manganese

An aqueous chlorogenic acid was prepared so as to have a concentration of 100 mM. Next, a 10 mM aqueous manganese (II) chloride (manufactured by nakalaitesque) was prepared, and mixed with an equal volume of the aqueous chlorogenic acid. The resulting mixture was allowed to stand at room temperature for 60 minutes. The enhancing activity for HGF production of this manganese-treated chlorogenic acid was assayed in the same manner as in Example 1. The manganese-treated chlorogenic acid was added in an amount of 1/1000 that of the medium (final concentration of chlorogenic acid: 50 µM). In addition, for the purpose of comparison, the activity of the chlorogenic acid without the manganese treatment was assayed in the same manner. The results are shown in Table 14. The enhancing activity for HGF production of chlorogenic acid was enhanced by the manganese treatment.

**Table 14**

| Sample | Amount of HGF Production (%) |
|---|---|
| 0 µM Chlorogenic Acid | 100 |
| 50 µM Chlorogenic Acid | 148 |
| 50 µM Manganese-Treated Chlorogenic Acid | 364 |
| (Here, the amount of HGF production in the control was 7.02 ng/ml.) | |

### Example 14 Enhancement of Enhancing Activity for HGF Production of Chlorogenic Acid by Copper

An aqueous chlorogenic acid was prepared so as to have a concentration of 100 mM. Next, a 10 mM aqueous copper (II) sulfate (manufactured by Nakarai Kagaku Yakuhin) was prepared, and mixed with an equal volume of the aqueous chlorogenic acid. The resulting mixture was allowed to stand at room temperature for 60 minutes. The enhancing activity for HGF production of this copper-treated chlorogenic acid was assayed in the same manner as in Example 1. The copper-treated chlorogenic acid was added in an amount of 1/1000 that of the medium (final concentration of chlorogenic acid: 50 µM). In addition, for the purpose of comparison, the activity of the chlorogenic acid without the copper treatment was assayed in the same manner. The results are shown in Table 15. The enhancing activity for HGF production of chlorogenic acid was enhanced by the copper treatment.

**Table 15**

| Sample | Amount of HGF Production (%) |
|---|---|
| 0 µM Chlorogenic Acid | 100 |
| 50 µM Chlorogenic Acid | 148 |
| 50 µM Copper-Treated Chlorogenic Acid | 293 |
| (Here, the amount of HGF production in the control was 7.02 ng/ml.) | |

### Example 15 Enhancement of Enhancing Activity for HGF Production of Oxidation-Treated Product of Chlorogenic Acid

Chlorogenic acid was dissolved in 100 mM sodium carbonate buffer (pH 9) so as to have a concentration of 100 mM. Air was blown into the resulting solution via a peristaltic pump for 12 hours, to prepare an oxidation-treated product of chlorogenic acid. The enhancing activity for HGF production of this oxidation-treated chlorogenic acid was assayed in the same manner as in Example 1. The oxidation-treated chlorogenic acid was added so as to have a concentration of 0, 1, 10, 100 or 1000 µM when calculated as chlorogenic acid. Incidentally, in the case where the final concentration of the oxidation-treated chlorogenic acid was 0 µM, a given amount of an aqueous solution obtained by oxidation treatment in the absence of chlorogenic acid was added to the medium. In addition, for the purpose of comparison, the activity of the chlorogenic acid without the oxidation treatment was assayed in the same manner. The results are shown in Table 16. The enhancing activity for HGF production of chlorogenic acid was enhanced by the oxidation treatment.

**Table 16**

| Concentration (µM) | | 0 | 1 | 10 | 100 | 1000 |
|---|---|---|---|---|---|---|
| Without Oxidation Treatment | Amount of HGF Production (%) | 100 | 105 | 122 | 302 | 624 |
| Oxidation Treatment | Amount of HGF Production (%) | 100 | 198 | 541 | 704 | 687 |
| (Here, the amount of HGF production in the control was 5.16 ng/ml.) | | | | | | |

### Example 16 Enhancement of Enhancing Activity for HGF Production of Oxidation-Treated Product of Caffeic Acid

Caffeic acid was dissolved in 100 mM sodium carbonate buffer (pH 9) so as to have a concentration of 100 mM. Air was blown into the resulting solution via a peristar pump for 12 hours, to prepare an oxidation-treated product of caffeic acid. The enhancing activity for HGF production of this oxidation-treated caffeic acid was assayed in the same manner as in Example 1. The oxidation-treated chlorogenic acid was added so as to have a concentration of 0, 1, 10, 100 or 1000 µM when calculated as caffeic acid. In addition, for the purpose of comparison, the activity of the caffeic acid without the oxidation treatment was assayed in the same manner. The results are shown in Table 17. The enhancing activity for HGF production of caffeic acid was enhanced by the oxidation treatment.

**Table 17**

| Concentration (µM) | | 0 | 1 | 10 | 100 | 1000 |
|---|---|---|---|---|---|---|
| Without Oxidation Treatment | Amount of HGF Production (%) | 100 | 85 | 117 | 196 | 92 |
| Oxidation Treatment | Amount of HGF Production (%) | 100 | 307 | 353 | 411 | 555 |
| (Here, the amount of HGF production in the control was 6.27 ng/ml.) | | | | | | |

### Example 17 Enhancement of Enhancing Activity for NGF Production of Chlorogenic Acid by Iron (1)

An aqueous solution of chlorogenic acid (manufactured by Tokyo Kasei Kogyo) was prepared so as to have a concentration of 100 mM. Next, a 10 mM aqueous ferric chloride (manufactured by Nakarai Kagaku Yakuhin) was prepared, and mixed with an equal volume of the aqueous solution of chlorogenic acid. The resulting mixture was allowed to stand at room temperature for 60 minutes, to prepare an iron-treated chlorogenic acid. The effect of this iron-treated chlorogenic acid on NGF production was assayed by the method described below.

L-M cells (ATCC CCL-1.2) from murine fibroblasts were suspended in an M199 medium (manufactured by ICN) containing 0.5% bactopeptone (manufactured by Gibco BRL) so as to have a concentration of 1.5 × 10⁵ cells/ml. The suspension was put in a 96-well plate in an amount of 0.1 ml each well, and the cells were aseptically cultured. After culturing the cells for 3 days, the medium was removed therefrom, and exchanged with an M199 medium containing 0.5% bovine serum albumin (manufactured by Sigma). To this was added the above iron-treated chlorogenic acid in an amount of 1/100 that of the medium (final concentration of chlorogenic acid: 500 µM), and the cells were cultured for 20 hours. After the termination of the culture, the NGF concentration in the culture medium was determined by an enzyme immunoassay method (NGF Emax Immuno Assay System, manufactured by Promega). Incidentally, in the case where the final concentration of the iron-treated chlorogenic acid was 0 µM, a given amount of an aqueous solution obtained by an iron treatment in the absence of chlorogenic acid was added to the medium. In addition, for the purpose of comparison, the activity of the chlorogenic acid without the iron treatment was assayed in the same manner. As described above, that with addition of a given amount of distilled water alone (final concentration of chlorogenic acid without the iron treatment: 0 µM) was used as the control. The amount of NGF production was expressed as the relative value (%) when the NGF concentration in the cell culture medium of the control was defined as 100%. The results are shown in Table 18. The experiment was carried out twice, and an average value was taken. The enhancing activity for NGF production of chlorogenic acid was enhanced by the iron treatment.

**Table 18**

| Concentration (µM) | | 0 | 500 |
|---|---|---|---|
| Without Iron Treatment | Amount of NGF Production (%) | 100 | 351.1 |
| Iron Treatment | Amount of NGF Production (%) | 98.8 | 476.7 |
| (Here, the amount of NGF production in the control was 0.174 ng/ml.) | | | |

### Example 18 Enhancement of Enhancing Activity for NGF Production of Chlorogenic Acid by Iron (2)

An aqueous chlorogenic acid was prepared so as to have a concentration of 100 mM. Next, a 5, 10 or 20 mM aqueous ferric chloride was prepared, and mixed with an equal volume of the aqueous chlorogenic acid. The resulting mixture was allowed to stand at room temperature for 60 minutes. The enhancing activity for NGF production of this iron-treated chlorogenic acid was assayed in the same manner as in Example 17. The iron-treated chlorogenic acid was added in an amount of 1/100 that of the medium (final concentration of chlorogenic acid: 500 µM). In addition, for the purpose of comparison, the activity of the chlorogenic acid without the iron treatment was assayed in the same manner. The results are shown in Table 19. Iron enhanced the enhancing activity for NGF production of chlorogenic acid in a concentration-dependent manner.

**Table 19**

| Concentration (µM) | 0 | 500 | 500 | 500 | 500 |
|---|---|---|---|---|---|
| Iron Concentration (µM) | 0 | 0 | 25 | 50 | 100 |
| Amount of NGF Production (%) | 100 | 351.1 | 355.0 | 441.6 | 472.6 |
| (Here, the amount of NGF production in the control was 0.176 ng/ml.) | | | | | |

### Example 19 Enhancement of Enhancing Activity for NGF Production of Chlorogenic Acid by Manganese (1)

An aqueous chlorogenic acid was prepared so as to have a concentration of 25, 50 or 100 mM. Next, a 10 mM aqueous manganese (II) chloride (manufactured by nakalaitesque) was prepared, and mixed with an equal volume of the aqueous chlorogenic acid. The resulting mixture was allowed to stand at room temperature for 60 minutes. The enhancing activity for NGF production of this manganese-treated chlorogenic acid was assayed in the same manner as in Example 17. The manganese-treated chlorogenic acid was added in an amount of 1/100 that of the medium (final concentration of chlorogenic acid: 125, 250 or 500 µM). In addition, for the purpose of comparison, the activity of the chlorogenic acid without the manganese treatment was assayed in the same manner. The results are shown in Table 20. The enhancing activity for NGF production of chlorogenic acid was enhanced by the manganese treatment.

**Table 20**

| Concentration (µM) | | 0 | 125 | 250 | 500 |
|---|---|---|---|---|---|
| Without Manganese Treatment | Amount of NGF Production (%) | 100 | 124.6 | 308.5 | 382.2 |
| Manganese Treatment | Amount of NGF Production (%) | 94.0 | 495.3 | 691.0 | 877.4 |
| (Here, the amount of NGF production in the control was 0.145 ng/ml.) | | | | | |

### Example 20 Enhancement of Enhancing Activity for NGF Production of Chlorogenic Acid by Manganese (2)

An aqueous chlorogenic acid was prepared so as to have a concentration of 100 mM. Next, 2.5, 5 or 10 mM aqueous manganese (II) chloride was prepared, and mixed with an equal volume of the aqueous chlorogenic acid. The resulting mixture was allowed to stand at room temperature for 60 minutes. The enhancing activity for NGF production of this manganese-treated chlorogenic acid was assayed in the same manner as in Example 17. The manganese-treated chlorogenic acid was added in an amount of 1/100 that of the medium (final concentration of chlorogenic acid: 500 µM). In addition, for the purpose of comparison, the activity of the chlorogenic acid without the manganese treatment was assayed in the same manner. The results are shown in Table 21. Manganese enhanced the enhancing activity for NGF production of chlorogenic acid in a concentration-dependent manner.

**Table 21**

| Concentration (µM) | 0 | 500 | 500 | 500 | 500 |
|---|---|---|---|---|---|
| Manganese Concentration (µM) | 0 | 0 | 12.5 | 25 | 50 |
| Amount of NGF Production (%) | 100 | 328.5 | 567.5 | 647.6 | 661.6 |
| (Here, the amount of NGF production in the control was 0.186 ng/ml.) | | | | | |

### Example 21 Enhancement of Enhancing Activity for NGF Production of Caffeic Acid by Manganese

An aqueous solution of caffeic acid (manufactured by Sigma) was prepared so as to have a concentration of 12.5, 25 or 50 mM. Next, a 10 mM aqueous manganese (II) chloride was prepared, and mixed with an equal volume of the aqueous caffeic acid. The resulting mixture was allowed to stand at room temperature for 60 minutes. The enhancing activity for NGF production of this manganese-treated caffeic acid was assayed in the same manner as in Example 17. The manganese-treated caffeic acid was added in an amount of 1/100 that of the medium (final concentration of caffeic acid: 62.5, 125 or 250 µM). In addition, for the purpose of comparison, the activity of the caffeic acid without the manganese treatment was assayed in the same manner. The results are shown in Table 22. The enhancing activity for NGF production of caffeic acid was enhanced by the manganese treatment.

**Table 22**

| Concentration (µM) | | 0 | 62.5 | 125 | 250 |
|---|---|---|---|---|---|
| Without Manganese Treatment | Amount of NGF Production (%) | 100 | 97.8 | 199.5 | 221.2 |
| Manganese Treatment | Amount of NGF Production (%) | 96.4 | 188.2 | 235.2 | 267.4 |
| (Here, the amount of NGF production in the control was 0.253 ng/ml.) | | | | | |

### Example 22 Enhancement of Enhancing Activity for NGF Production of Oxidation-Treated Product of Chlorogenic acid

Chlorogenic acid was dissolved in 100 mM sodium carbonate buffer (pH 9) so as to have a concentration of 100 mM. Air was blown into the resulting solution via a peristar pump for 12 hours, to prepare an oxidation-treated product of chlorogenic acid. The enhancing activity for NGF production of this oxidation-treated chlorogenic acid was assayed in the same manner as in Example 17. The oxidation-treated chlorogenic acid was added so as to have a concentration of 0, 250, 500 or 1000 µM when calculated as chlorogenic acid. Incidentally, in the case where the final concentration of the oxidation-treated chlorogenic acid was 0 µM, a given amount of an aqueous solution obtained by oxidation treatment in the absence of chlorogenic acid was added to the medium. In addition, for the purpose of comparison, the activity of the chlorogenic acid without the oxidation treatment was assayed in the same manner. The results are shown in Table 23. The enhancing activity for NGF production of chlorogenic acid was enhanced by the oxidation treatment.

**Table 23**

| Concentration (µM) | | 0 | 250 | 500 | 1000 |
|---|---|---|---|---|---|
| Without Oxidation Treatment | Amount of NGF Production (%) | 100 | 192.8 | 231.9 | 256.2 |
| Oxidation Treatment | Amount of NGF Production (%) | 100 | 211.0 | 368.7 | 642.7 |
| (Here, the amount of NGF production in the control was 0.166 ng/ml.) | | | | | |

### Example 23 Enhancement of Enhancing Activity for NGF Production of Oxidation-Treated Product of Caffeic Acid

Caffeic acid was dissolved in 100 mM sodium carbonate buffer (pH 9) so as to have a concentration of 100 mM. Air was blown into the resulting solution via a peristar pump for 12 hours, to prepare an oxidation-treated product of caffeic acid. The enhancing activity for NGF production of this oxidation-treated caffeic acid was assayed in the same manner as in Example 22. The oxidation-treated caffeic acid was added so as to have a concentration of 0, 250, 500 or 1000 µM when calculated as caffeic acid. In addition, for the purpose of comparison, the activity of the caffeic acid without the oxidation treatment was assayed in the same manner. The results are shown in Table 24. The enhancing activity for NGF production of caffeic acid was enhanced by the oxidation treatment.

**Table 24**

| Concentration (µM) | | 0 | 250 | 500 | 1000 |
|---|---|---|---|---|---|
| Without Oxidation Treatment | Amount of NGF Production (%) | 100 | 227.8 | 287.9 | 269.3 |
| Oxidation Treatment | Amount of NGF Production (%) | 100 | 275.3 | 449.5 | 655.2 |
| (Here, the amount of NGF production in the control was 0.218 ng/ml.) | | | | | |

### Example 24 Preparation and Enhancing Activity for NGF Production of Caffeic Acid Ester

### (1) Preparation of Caffeic Acid Methyl Ester

The amount 0.1 g of caffeic acid was dissolved in methanol containing 10% sulfuric acid, and heated in the presence of anhydrous sodium sulfate at 100°C for 1 hour. Anhydrous sodium sulfate was filtered off from the reaction solution, and the filtrate was concentrated to dryness with a rotary evaporator. Next, the reaction product was purified by reversed-phase chromatography. The conditions are shown below. The column used was TSK gel ODS-80Ts (diameter: 21.5 mm, length: 30 cm, manufactured by Tosoh Corporation). The elution ratio of Solvent A (mixture of distilled water and acetonitrile in a volume ratio of 2:3, containing 0.1% trifluoroacetic acid) and Solvent B (mixture of distilled water and acetonitrile in a volume ratio of 1:4, containing 0.1% trifluoroacetic acid) was such that the ratio of Solvent B was increased linearly from 25% to 45% from 0 to 30 minutes, and the ratio of Solvent B was retained at 45% for the subsequent 15 minutes. The elution rate was 5 ml/minute, and the detection was carried out at 215 nm. The fractions including a peak at a retention time of 19.8 minutes were collected, and concentrated to dryness, to give 3.7 mg of a compound. The resulting compound was analyzed by NMR. As a result, the compound was confirmed to be caffeic acid methyl ester.

The determination results of the NMR spectrum and the mass spectrum are shown below.
¹H-NMR: δ 3.67(3H, s, OCH₃), 6.26(1H, d, J=16.0 Hz, 2'-H), 6.74(1H, d, J=8.0 Hz, 5-H), 6.99(1H, dd, J=2.0, 8.0 Hz, 6-H), 7.04(1H, d, J=2.0 Hz, 2-H), 7.47(1H, d, J=16.0 Hz, 3'-H), 9.16(1H, s, 3-OH), 9.63(1H, s, 4-OH)

Here, the sample was dissolved in deuterated dimethyl sulfoxide, and the chemical shift of the residual dimethyl sulfoxide was expressed as 2.49 ppm.
FAB-MS : m/z 195 (M+H)⁺ (Here, glycerol was used as the matrix.)

### (2) Preparation of Caffeic Acid Ethyl Ester

The amount 0.1 g of caffeic acid was dissolved in ethanol containing 10% sulfuric acid, and the resulting solution was subjected to a heating reaction and a purification operation in the same manner as in item (1) of Example 24. Fraction including a peak at a retention time of 23.8 minutes on reversed-phase chromatography was collected, and concentrated to dryness, to give 2.7 mg of a compound. The resulting compound was analyzed by NMR. As a result, the compound was confirmed to be caffeic acid ethyl ester.

The determination results of the NMR spectrum and the mass spectrum are shown below.
¹H-NMR: δ 1.22 (3H, t, J=7.0 Hz, 2"-H), 4.14 (2H, q, J=7.0 Hz, 1"-H), 6.24 (1H, d, J=16.0 Hz, 2'-H), 6.74 (1H, d, J=8.0 Hz, 5-H), 6.99 (1H, dd, J=2.0, 8.0 Hz, 6-H), 7.03 (1H, d, J=2.0 Hz, 2-H), 7.45 (1H, d, J=16.0 Hz, 3'-H), 9.15 (1H, s, 3-OH), 9.62 (1H, s, 4-OH)

Here, the sample was dissolved in deuterated dimethyl sulfoxide, and the chemical shift of the residual dimethyl sulfoxide was expressed as 2.49 ppm.
FAB-MS : m/z 209 (M+H)⁺ (Here, glycerol was used as the matrix.)

### (3) Enhancing Activities for NGF Production of Caffeic Acid Methyl Ester and Caffeic Acid Ethyl Ester

The enhancing activities for NGF production of caffeic acid methyl ester prepared and caffeic acid ethyl ester prepared in items (1) and (2) of Example 24 were, respectively, assayed in the same manner as in Example 17.

Caffeic acid methyl ester was added so as to have a final concentration of 0, 0.0625 or 0.125 mg/ml, while caffeic acid ethyl ester was added so as to have a final concentration of 0 or 0.0625 mg/ml. The results are shown in Table 25. Caffeic acid methyl ester and caffeic acid ethyl ester enhanced NGF production in the L-M cells.

**Table 25**

| Sample | Concentration (mg/ml) | Amount of NGF Production (%) |
|---|---|---|
| Caffeic Acid Methyl Ester | 0 | 100 |
| | 0.0625 | 824.4 |
| | 0.125 | 889.6 |
| Caffeic Acid Ethyl Ester | 0 | 100 |
| | 0.0625 | 1279.1 |
| (Here, the amount of NGF production in the control was 0.109 ng/ml.) | | |

### Example 25 Enhancing Activity for NGF Production of Caffeic Acid Methyl Ester

### (1) Preparation of XAD-2-treated Product of Water-extracted Low Molecular Weight Fraction from Root Portions of Angelica keiskei koidz.

The amount 5.8 kg of powder of root portions of dried *Angelica keiskei* koidz, was extracted with 24 L of ethyl acetate at room temperature for 3 hours. The residue after the suction filtration was extracted with 18 L of ethanol overnight at room temperature. Next, the residue after the suction filtration was extracted with 52 L of distilled water at 60°C for 3 hours. A liquid portion resulting from removal of the solid residue was concentrated with a rotary evaporator. A 2.5-fold volume of ethanol was added to the concentrate, and the resulting mixture was allowed to stand overnight at 4°C. Subsequently, the mixture was fractionated into a liquid portion and precipitates by suction filtration, and the liquid portion was concentrated to dryness with a rotary evaporator, to give a water-extracted, low-molecular weight fraction from root portions of *Angelica keiskei* koidz. Next, the water-extracted, low-molecular weight fraction from root portions of *Angelica keiskei* koidz. was applied to Amberlite XAD-2 (manufactured by Organo; resin amount: 2 L). A non-adsorbed substance was sufficiently washed out with 30 L of distilled water, and an adsorbed substance was then eluted with 16 L of methanol. The methanol eluate was concentrated to dryness with a rotary evaporator, to give an XAD-2-treated product of the water-extracted, low-molecular weight fraction from root portions of *Angelica keiskei* koidz.

### (2) Enhancement of NGF Production by XAD-2-Treated Product of Water-Extracted, Low-Molecular Weight Fraction from Root Portions of Angelica keiskei koidz.

The enhancing activity for NGF production of the XAD-2-treated product of the water-extracted low molecular weight fraction from root portions of *Angelica keiskei* koidz. prepared in item (1) of Example 25 was assayed in the same manner as in Example 17. The XAD-2-treated, water-extracted, low-molecular weight fraction from root portions of *Angelica keiskei* koidz. was added so as to have a concentration of 0, 0.85, 1.7 or 3.4 mg/ml. The XAD-2-treated product of the water-extracted, low-molecular weight fraction from root portions of *Angelica keiskei* koidz. enhanced NGF production in the L-M cells in a concentration-dependent manner, as shown in Table 26.

**Table 26**

| XAD-2-treated Product of Water-Extracted, Low-Molecular Weight Fraction from Root Portions of *Angelica keiskei* koidz. (mg/ml) | Amount of NGF Production (%) |
|---|---|
| 0 | 100 |
| 0.85 | 655.3 |
| 1.7 | 858.8 |
| 3.4 | 1127.6 |
| (Here, the amount of NGF production in the control was 0.074 ng/ml.) | |

### (3) Fractionation of XAD-2-Treated Product of Water-Extracted, Low-Molecular Weight Fraction from Root Portions of Angelica keiskei koidz. by Cosmosil 140 Chromatography

An active component of the XAD-2-treated product of the water-extracted low-molecular weight fraction from root portions of *Angelica keiskei* koidz. prepared in item (1) of Example 25 was fractionated by reversed-phase chromatography. The conditions are shown below. The resin used was Cosmosil 140 C18-OPN (manufactured by nakalaitesque; resin amount: 400 ml). The XAD-2-treated product of the water-extracted, low-molecular weight fraction from root portions of *Angelica keiskei* koidz. was subjected to the chromatography, and elution was carried out sequentially using 1 L each of distilled water, a 20% aqueous acetonitrile, a 25% aqueous acetonitrile, a 40% aqueous acetonitrile and methanol as a developing solvent. Each eluted fraction was concentrated under reduced pressure.

### (4) Enhancement of NGF Production by Cosmosil 140 Chromatography-Fractionated Product of XAD-2-Treated Product of Water-Extracted, Low-Molecular Weight Fraction from Root Portions of Angelica keiskei koidz.

The enhancing activity for NGF production of the Cosmosil 140 chromatography-fractionated product prepared in item (3) of Example 25 was assayed in the same manner as in Example 17. As a result, it was clarified that the 20% aqueous acetonitrile-eluted fraction, the 25% aqueous acetonitrile-eluted fraction and the 40% aqueous acetonitrile-eluted fraction had an enhancing activity for NGF production. The results are shown in Table 27.

**Table 27**

| Fraction | Concentration (mg/ml) | Amount of NGF Production (%) |
|---|---|---|
| 20% Aqueous Acetonitrile-Eluted Fraction | 4.05 | 301.0 |
| | 8.1 | 436.7 |
| | 16.2 | 1263.3 |
| 25% Aqueous Acetonitrile-Eluted Fraction | 0.7 | 161.7 |
| | 1.5 | 1028.8 |
| | 2.8 | 2110.4 |
| 40% Aqueous Acetonitrile-Eluted Fraction | 0.575 | 465.3 |
| | 1.15 | 653.1 |
| | 2.3 | 1226.2 |
| (Here, the amounts of NGF production in the controls were 0.074 ng/ml for the 20% acetonitrile-eluted fraction, and 0.087 ng/ml for the 25% or 40% acetonitrile-eluted fraction.) | | |

### (5) Fractionation of 25% Aqueous Acetonitrile-Eluted Fraction on Cosmosil 140 by ODS-80 Ts Chromatography

An active component of the 25% aqueous acetonitrile-eluted fraction on the Cosmosil 140 prepared in item (3) of Example 25 was fractionated by reversed-phase chromatography. The conditions are shown below. The column used was TSK gel ODS-80 Ts (diameter: 21.5 mm, length: 30 cm, manufactured by Tosoh Corporation). The elution ratio of Solvent A (distilled water) and Solvent B (mixture of distilled water and acetonitrile in a volume ratio of 1:1) was such that the ratio of Solvent B was increased linearly from 25 to 100% from 0 to 120 minutes, the ratio of Solvent B was retained at 100% for the next 20 minutes, and the ratio of Solvent B was finally decreased to 25% and retained thereat for 20 minutes. The elution rate was 5 ml/minute, and the detection was carried out at 235 nm. Fractions were collected using UV absorption as an index.

### (6) Enhancement of NGF Production by ODS-80 Ts Chromatography-Fractionated Product of 25% Aqueous Acetonitrile-Eluted Fraction on Cosmosil 140

The activity of the ODS-80 Ts chromatography-fractionated product of the 25% aqueous acetonitrile-eluted fraction on the Cosmosil prepared in item (5) of Example 25 was assayed in the same manner as in Example 17. As a result, it was clarified that many fractions had an enhancing activity for NGF production. The results are shown in Table 28.

**Table 28**

| Fractionated Fraction (Detected Peak: minute) | Concentration (mg/ml) | Amount of NGF Production (%) |
|---|---|---|
| 1 (46.0, 47.2, 49.0) | 2.00 | 250.8 |
| 2 (53.2) | 2.00 | 275.2 |
| 3 (54.0) | 2.00 | 318.7 |
| 4 (54.9, 55.5, 56.4) | 2.00 | 293.0 |
| 5 (57. 6) | 2.00 | 320.5 |
| 6 (58.6) | 2.00 | 297.8 |
| 7 (59.3) | 2.00 | 324.8 |
| 8 (60.5) | 2.00 | 324.8 |
| 9 (61.3) | 2.00 | 402.8 |
| 10 (62.2) | 2.00 | 565.5 |
| 11 (63.0) | 2.00 | 616.9 |
| 12 (64.1) | 2.00 | 575.4 |
| 13 (66.9) | 2.00 | 805.3 |
| 14 (68.4) | 2.00 | 819.6 |
| 15 (69.2) | 2.00 | 510.2 |
| 16 (70.3) | 1.00 | 389.2 |
| 17 (71.3, 71.9) | 1.00 | 609.1 |
| 18 (73.0, 73.8, 74.9) | 0.50 | 1002.5 |
| 19 (75.4) | 0.50 | 851.3 |
| 20 (76.5) | 1.00 | 838.5 |
| 21 (77.7) | 1.00 | 249.4 |
| 22 (79.6, 80.5) | 0.50 | 234.3 |
| 23 (82.4) | 0.25 | 359.1 |
| 24 (84.1) | 0.25 | 285.8 |
| 25 (85.5) | 0.0625 | 359.1 |
| 26 (86.9, 87.5) | 0.10 | 411.6 |
| 27 (89.1) | 0.50 | 443.3 |
| 28 (91.4) | 0.05 | 1058.1 |
| 29 (92.8) | 0.20 | 672.0 |
| 30 (93.8, 95.8, 97.5, 100.0, 100.6) | 0.50 | 593.6 |
| (Here, the amounts of NGF production in the controls were 0.355 ng/ml for fractions 1 - 9, 0.382 ng/ml for fractions 10 - 18, 0.415 ng/ml for fractions 19 - 27, and 0.450 ng/ml for fractions 28 - 30.) | | |

### (7) Fractionation of Fraction 18 by Reversed-Phase Chromatography

Fraction 18 (fraction including a detected peak at a retention time of 73.0, 73.8 or 74.9 minutes), which was confirmed to have a strong activity in item (6) of Example 25, was further fractionated by reversed-phase chromatography. The conditions are shown below. The column used was TSK gel ODS-80TsQA (diameter: 4.6 mm, length: 25 cm, manufactured by Tosoh Corporation). The elution ratio of Solvent A (distilled water containing 0.1% trifluoroacetic acid) and Solvent B (mixture of distilled water and acetonitrile in a volume ratio of 1:1, containing 0.1% trifluoroacetic acid) was such that the ratio of Solvent B was retained at 50% for 20 minutes. The elution rate was 1 ml/minute, and the detection was carried out at 235 nm. Fractions were collected using UV absorption as an index.

### (8) Enhancement of NGF Production by ODS-80 TsQA Chromatography-Fractionated Product of Fraction 18

The activity of the fraction, which was fractionated by TSK gel ODS-80 TsQA chromatography in item (7) of Example 25, was assayed in the same manner as in Example 17. As a result, it was clarified that fractions including a detected peak at a retention time of 9.3, 10.1, 10.6, 10.9, 11.2, 12.0, 12.8, 13.3, 14.0, 15.2, 16.1 or 18.6 minutes had an enhancing activity for NGF production. The results are shown in Table 29.

**Table 29**

| Fractionated Fraction (Detected Peak: minute) | Concentration (mg/ml) | Amount of NGF Production (%) |
|---|---|---|
| 18-1 (9.3) | 0.20 | 1489.6 |
| 18-2 (10.1) | 0.50 | 2222.9 |
| 18-3 (10.6) | 0.25 | 3039.6 |
| 18-4 (10.9) | 0.0375 | 2510.4 |
| 18-5 (11.2) | 0.125 | 610.4 |
| 18-6 (12.0) | 0.50 | 560.4 |
| 18-7 (12.8) | 0.50 | 681.3 |
| 18-8 (13.3) | 0.40 | 339.6 |
| 18-9 (14.0) | 1.00 | 410.4 |
| 18-10 (15.2) | 1.00 | 2510.7 |
| 18-11 (16.1) | 1.00 | 3360.7 |
| 18-12 (18.6) | 1.00 | 1189.3 |
| (Here, the amounts of NGF production in the controls were 0.027 ng/ml for 18-1 - 18-9, and 0.015 ng/ml for 18-10 - 18-12.) | | |

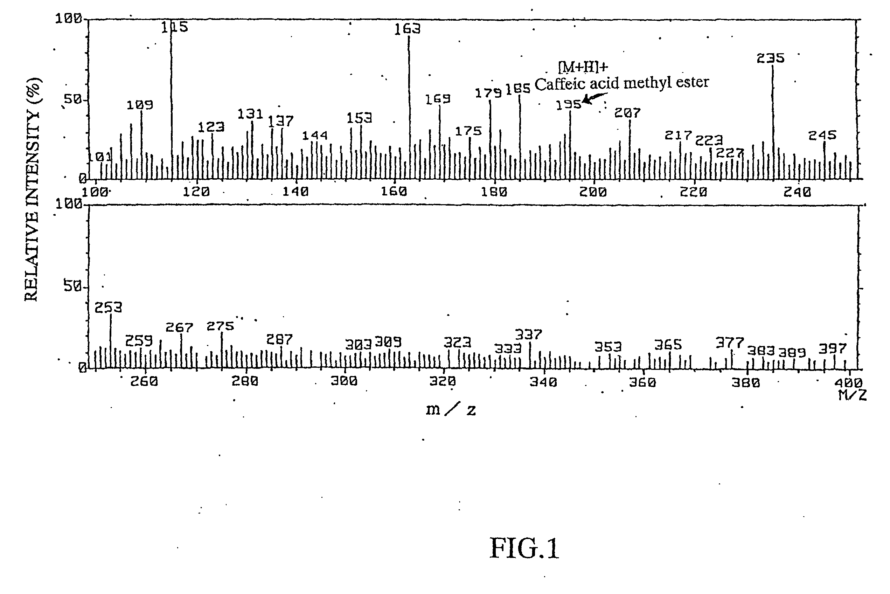

The mass spectrum (MS) of the Fraction 18-3 from root portions of *Angelica keiskei* koidz., which was prepared in item (7) of Example 25 and confirmed to have the activity in item (8) of Example 25, was determined by a mass spectrometer (DX302, manufactured by JEOL LTD.) using the FAB-MS method. Glycerol was used as the matrix. As a result, a peak was detected at m/z 195 (M+H)⁺. The MS spectrum of the Fraction 18-3 from root portions of *Angelica keiskei* koidz. is shown in Figure 1. In Figure 1, the axis of abscissas is the value of m/z, and the axis of ordinates is the relative intensity.

### (9) ¹H-NMR Analysis of Fraction 18-3

Various NMR spectra of the Fraction 18-3 from root portions of *Angelica keiskei* koidz., which was prepared in item (7) of Example 25 and confirmed to have the activity in item (8) of Example 25, were determined using a nuclear magnetic resonance (NMR) spectrometer (JNM-A500, manufactured by JEOL LTD.), and the structure was analyzed. The signals of NMR are shown below.
¹H-NMR: δ 3.68 (3H, s, OCH₃), 6.25 (1H, d, J = 16.0 Hz, 2'-H), 6.75 (1H, d, J = 8.0 Hz, 5-H), 6.98 (1H, dd, J = 2.0, 8.0 Hz, 6-H), 7.03 (1H, d, J = 2.0, 2-H), 7.46 (1H, d, J = 16.0 Hz, 3'-H), 9.10 (1H, s, 3-OH), 9.56 (1H, s, 4-OH)

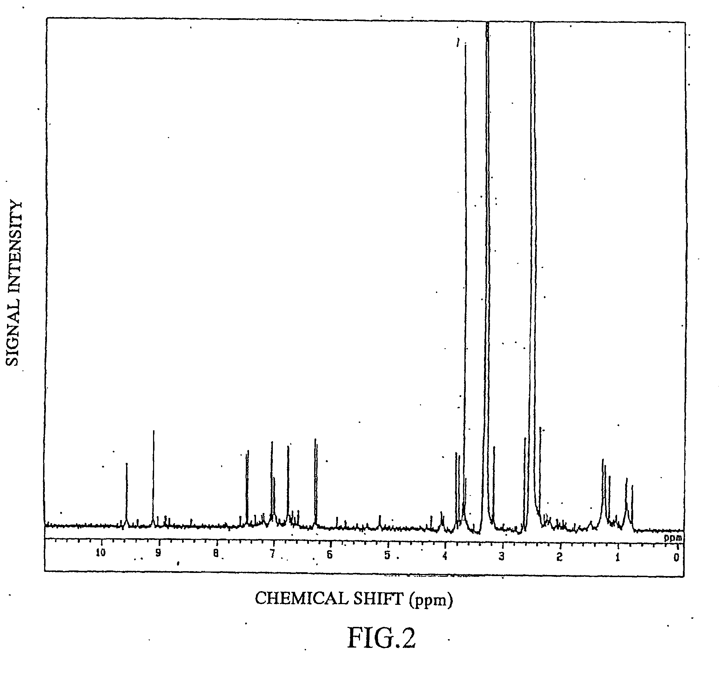

Here, the sample was dissolved in deuterated dimethyl sulfoxide, and the chemical shift of the residual dimethyl sulfoxide was expressed as 2.49 ppm. The ¹H-NMR spectra of the Fraction 18-3 from root portions of *Angelica keiskei* koidz. is shown in Figure 2. In Figure 2, the axis of abscissas is the chemical shift (ppm), and the axis of ordinates is the intensity of signal.

### (10) Determination of Structure of Fraction 18-3

The mass spectrum analysis and the NMR spectrum analysis of the Fraction 18-3 from root portions of *Angelica keiskei* koidz., which was prepared in item (7) of Example 25 and confirmed to have the activity in item (8) of Example 25 were conducted. As a result, the active component was confirmed to be caffeic acid methyl ester (molecular weight: 194).

### Example 26 Enhancing Activity for NGF Production of Caffeic Acid Ethyl Ester

### (1) MS Analysis of Fraction 28

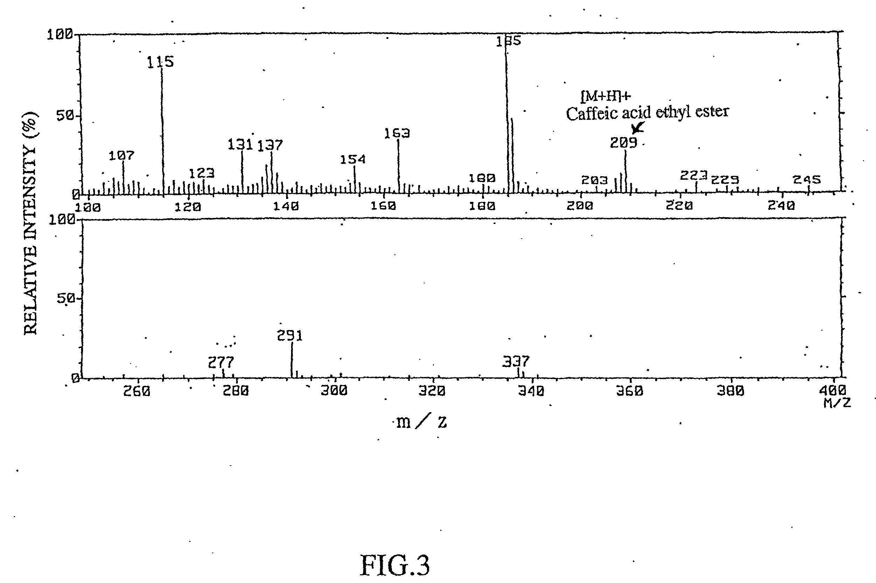

The mass spectrum (MS) of the Fraction 28 from root portions of *Angelica keiskei* koidz. (fraction including a detected peak at a retention time of 91.4 minutes), which was prepared in item (5) of Example 25 and confirmed to have the activity in item (6) of Example 25, was determined by a mass spectrometer (DX302, manufactured by JEOL LTD.) using the FAB-MS method. Glycerol was used as the matrix. As a result, a peak was detected at m/z 209 (M+H)⁺. The MS spectrum of the Fraction 28 from root portions of *Angelica keiskei* koidz. is shown in Figure 3. In Figure 3, the axis of abscissas is the value of m/z, and the axis of ordinates is the relative intensity.

### (2) ¹H-NMR Analysis of Fraction 28

Various NMR spectra of the Fraction 28 from root portions of *Angelica* *keiskei* koidz., which was prepared in item (5) of Example 25 and confirmed to have the activity in item (6) of Example 25, were determined using a nuclear magnetic resonance (NMR) spectrometer (JNM-A500, manufactured by JEOL LTD.), and the structure was analyzed. The signals of NMR are shown below.
¹H-NMR : δ 1.23 (3H, t, J = 7.0 Hz, 2"-H), 4.14 (2H, q, J = 7.0 Hz, 1"-H), 6.24 (1H,d, J = 16.0 Hz, 2'-H), 6.74 (1H, d, J = 8.0 Hz, 5-H), 6.98 (1H, dd, J = 2.0, 8.0 Hz, 6-H), 7.03 (1H, d, J = 2.0 Hz, 2-H), 7.45 (1H, d, J = 16.0 Hz, 3'-H), 9.11 (1H, s, 3-OH), 9.57 (1H, s, 4-OH)

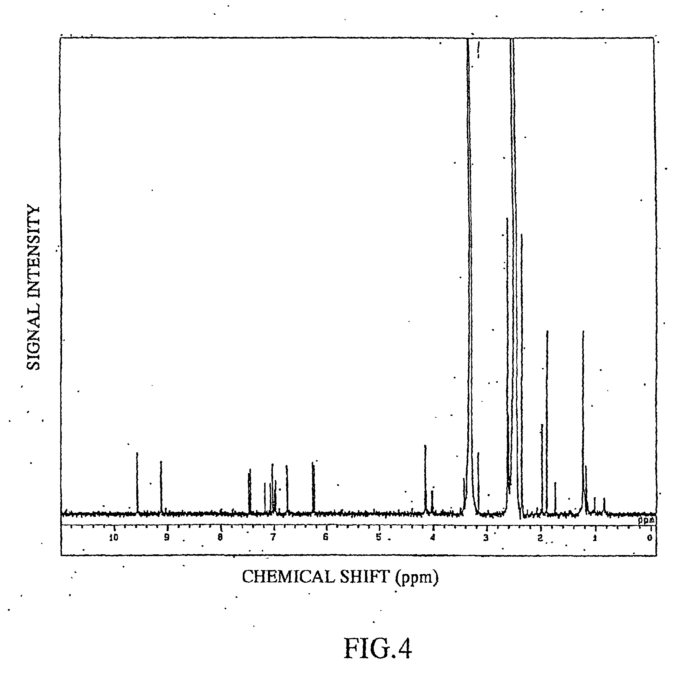

Here, the sample was dissolved in deuterated dimethyl sulfoxide, and the chemical shift of the residual dimethyl sulfoxide was expressed as 2.49 ppm. The ¹H-NMR spectra of the Fraction 28 from root portions of *Angelica keiskei* koidz. is shown in Figure 4. In Figure 4, the axis of abscissas is the chemical shift (ppm), and the axis of ordinates is the intensity of signal.

### (3) Determination of Structure of Fraction 28

The mass spectrum analysis and the NMR spectrum analysis of the Fraction 28 from root portions of *Angelica keiskei* koidz., which was prepared in item (5) of Example 25 and confirmed to have the activity in item (6) of Example 25 were conducted. As a result, the active component was confirmed to be caffeic acid ethyl ester (molecular weight: 208).

### Example 27 Enhancing Activity for NGF Production by Isoxanthohumol

(1) The amount 0.4 g of a fraction of xanthohumol derived from *Humulus lupulus* was dissolved in 3 ml of chloroform, and the resulting solution was subjected to silica gel chromatography. The elution was carried out stepwise by sequentially using chloroform (1000 ml), a chloroform/methanol mixture in a ratio of 50:1 (1000 ml) and a chloroform/methanol mixture in a ratio of 20:1 (1000 ml), and an 8 ml-aliquot was collected per fraction. The obtained fractions 67 to 100 were concentrated under reduced pressure, to give a fraction A of the fraction of xanthohumol derived from *Humulus lupulus.* The fraction A of the fraction of xanthohumol derived from *Humulus lupulus* was further purified by reversed-phase chromatography. The conditions are shown below. The column used was TSK gel ODS-80 Ts (diameter: 21.5 mm, length: 30 cm, manufactured by Tosoh Corporation). The elution ratio of Solvent A (mixture of distilled water and acetonitrile in a volume ratio of 3:2, containing 0.1% trifluoroacetic acid) and Solvent B (mixture of distilled water and acetonitrile in a volume ratio of 1:4, containing 0.1% trifluoroacetic acid) was such that the ratio of Solvent B was increased linearly from 50 to 100% from 0 to 60 minutes, the ratio of Solvent B was retained at 100% for the next 20 minutes, and the ratio of Solvent B was finally decreased to 50% and retained thereat for 20 minutes. The elution rate was 5 ml/minute, and the detection was carried out at 370 nm. Fractions were collected every minute. The fraction including a detected peak at a retention time of 42.5 minutes was concentrated to dryness, to prepare a high purity xanthohumol (19.4 mg).
(2) A solution prepared by dissolving 3.5 mg of xanthohumol prepared in item (1) of Example 27 in 0.75 ml of a dimethyl sulfoxide solution was added to 200 ml of 10 mM sodium acetate buffer (pH 5.5, containing 10% saccharose), and the resulting mixture was heated at 100°C for 2 hours. After cooling, the reaction solution was fractionated by reversed-phase chromatography. The conditions are shown below. The resin used was Cosmosil 140 C18-OPN (manufactured by nakalaitesque; resin amount: 20 ml). The reaction solution was applied to a column, and the elution was carried out sequentially using 50 ml each of distilled water, 20%, 30%, 40%, 50%, 60% and 70% aqueous acetonitrile solutions and methanol as a developing solvent. As a result, the 40% aqueous acetonitrile-eluted fraction was concentrated to dryness, to give a high purity isoxanthohumol (2.1 mg). Various NMR spectra of the resulting compound were determined and analyzed using a nuclear magnetic resonance (NMR) spectrometer (JNM-A500, manufactured by JEOL LTD.), and the structure was confirmed.
The determination results of the NMR spectra and the mass spectrum are shown below.
¹H-NMR : δ 1.52 (3H, s, 3"-CH₃), 1.57 (3H, s, 3"-CH₃), 2.54 (1H, dd, J = 3.0, 16.5 Hz, 3-H), 2.92 (1H, dd, J = 12.5, 16.5 Hz, 3-H), 3.09 (2H, d, J = 7.0 Hz, 1"-H), 3.68 (3H, s, 5-OCH₃), 5.07 (1H, t, J = 7.0 Hz, 2"-H), 5.30 (1H, dd, J = 3.0, 12.3 Hz, 2-H), 6.12 (1H, s, 6-H), 6.76 (2H, d, J = 8.5 Hz, 3'-H and 5'-H), 7.27 (2H, d, J = 8.5 Hz, 2'-H and 6'-H), 9.53 (1H, s, 4'-OH), 10.43 (1H, brs, 7-OH)
Here, the sample was dissolved in deuterated dimethyl sulfoxide, and the chemical shift of the residual dimethyl sulfoxide was expressed as 2.49 ppm.
FAB-MS : m/z 355 (M+H)⁺ (Here, glycerol was used as the matrix.)
(3) The enhancing activity for NGF production of isoxanthohumol prepared in item (2) of Example 27 was assayed in the same manner as in Example 17. The results are shown in Table 30. Isoxanthohumol was added so as to have a concentration of 0, 50, 100 or 200 µM. Isoxanthohumol enhanced NGF production in the L-M cells in a concentration-dependent manner.

**Table 30**

| Concentration (µM) | Amount of NGF Production (%) |
|---|---|
| 0 | 100 |
| 50 | 110.7 |
| 100 | 121.4 |
| 200 | 198.0 |
| (Here, the amount of NGF production in the control was 0.181 ng/ml.) | |

### Example 28

(1) The amount 0.4 g of a xanthohumol fraction was dissolved in 3 ml of chloroform, and the resulting solution was subjected to silica gel chromatography. The elution was carried out stepwise by sequentially using chloroform (1000 ml), a chloroform/methanol mixture in a ratio of 50:1 (1000 ml) and a chloroform/methanol mixture in a ratio of 20:1 (1000 ml). An 8 ml-aliquot was collected per fraction. The obtained fractions 67 to 100 were concentrated under reduced pressure, to give a fraction A of the fraction of xanthohumol derived from *Humulus lupulus*.
(2) The above-mentioned fraction A of the xanthohumol fraction was further subjected to purification and isolation by reversed-phase chromatography. The conditions are shown below. The column used was TSK gel ODS-80 Ts (diameter: 21.5 mm, length: 30 cm, manufactured by Tosoh Corporation). The elution ratio of Solvent A (mixture of distilled water and acetonitrile in a volume ratio of 3:2, containing 0.1% trifluoroacetic acid) and Solvent B (mixture of distilled water and acetonitrile in a volume ratio of 1:4, containing 0.1% trifluoroacetic acid) was such that the ratio of Solvent B was increased linearly from 50 to 100% from 0 to 60 minutes, the ratio of Solvent B was retained at 100% for the next 20 minutes, and the ratio of Solvent B was finally decreased to 50% and retained thereat for 20 minutes. The elution rate was 5 ml/minute, and the detection was carried out at 370 nm. Fractions were collected every minute.
(3) The enhancing activity for NGF production of the reversed-phase chromatography-fractionated fractions of the above fraction A of the xanthohumol fraction were assayed in the same manner as in Example 17. As a result, it was clarified that fractions including a detected peak at a retention time of 21.1, 22.2, 24.2, 25.7 or 28.6 minutes had an enhancing activity for NGF production. The results are shown in Table 31.

**Table 31**

| Fraction (Retention Time: minute) | Concentration (mg/ml) | Amount of NGF Production (%) |
|---|---|---|
| A-1 (21.1) | 0.025 | 92.4 |
| | 0.05 | 151.4 |
| | | |
| A-2 (22.2) | 0.05 | 109.5 |
| | 0.1 | 136.2 |
| | 0.2 | 208.6 |
| | | |
| A-3 (24.2) | 0.0125 | 134.3 |
| | 0.025 | 151.4. |
| | 0.05 | 202.9 |
| | 0.1 | 298.1 |
| | | |
| A-4 (25.7) | 0.025 | 197.1 |
| | 0.05 | 345.7 |
| | 0.1 | 416.2 |
| | | |
| A-5 (28.6) | 0.0125 | 145.7 |
| | 0.025 | 189.5 |
| | 0.05 | 229.5 |
| | 0.1 | 517.1 |
| (Here, the amount of NGF production in the control was 0.093 ng/ml.) | | |

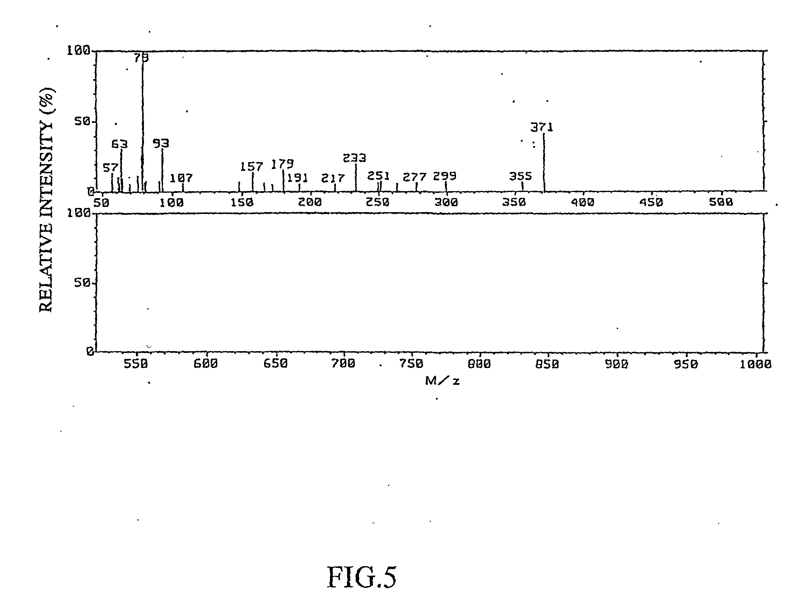

(4) The mass spectrum (MS) of the above Fraction A-5 (fraction including a detected peak at a retention time of 28.6 minutes) was determined by a mass spectrometer (DX302, manufactured by JEOL LTD.) using the FAB-MS method. Glycerol was used as the matrix. As a result, a peak was detected at m/z 371 (M+H)⁺. The MS spectrum of the Fraction A-5 from the xanthohumol fraction is shown in Figure 5. In Figure 5, the axis of abscissas is the value of m/z, and the axis of ordinates is the relative intensity.

Further, various NMR spectra were determined using a nuclear magnetic resonance (NMR) spectrometer (JNM-A500, manufactured by JEOL LTD.), and the structure was analyzed. As a result, the active component was confirmed to be a mixture of xanthohumol B (molecular weight: 370) and xanthohumol D (molecular weight: 370) (mixing ratio: 1:1.65). The signals of NMR are shown below.

### Xanthohumol B

¹H-NMR : δ 1.21 (3H, s, 6-H), 1.27 (3H, s, 6-H), 2.70 (2H, m, 4-H), 3.65 ((1H, m, 5-H), 3.87 (3H, s, 6-OCH₃), 6.00 (1H, s, 5-H), 6.80 (2H, m, 3-H and 5-H), 7.55 (2H, m, 2-H and 6-H), 7.70 (1H, m, β-H), 7.75 (1H, m, α-H), 10.12 (1H, s, 4-OH), 14.18 (1H, s, 2-OH)

### Xanthohumol D

¹H-NMR : δ 1.71 (3H, s, 5-H), 2.60 (2H, m, 1-H), 3.85 (3H, s, 6-OCH₃), 4.20 (1H, m, 2-H), 4.58 (1H, s, 4-H), 4.61 (1H, s, 4-H), 6.04 (1H, s, 5-H), 6.80 (2H, m, 3-H and 5-H), 7.55 (2H, m, 2-H and 6-H), 7.70 (1H, m, β-H), 7.75(1H, m, α-H), 10.09 (1H, s, 4-OH), 10.58 (1H, s, 4-OH), 14.69 (1H, s, 2-OH)

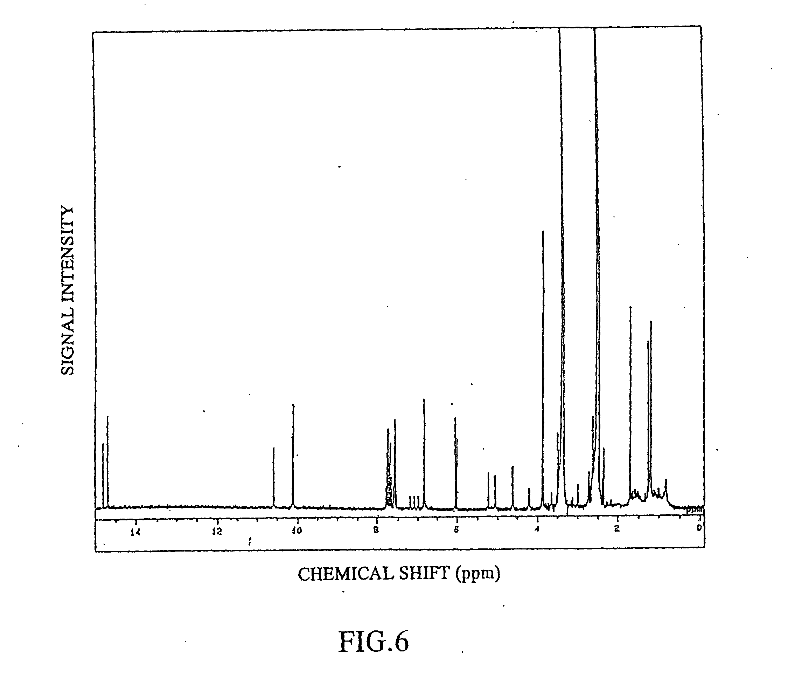

Here, the sample was dissolved in deuterated dimethyl sulfoxide, and the chemical shift of the residual dimethyl sulfoxide was expressed as 2.49 ppm. The ¹H-NMR spectrum of the Fraction A-5 derived from the xanthohumol fraction is shown in Figure 6. In Figure 6, the axis of abscissas is the chemical shift (ppm), and the axis of ordinates is the intensity of signal.

### INDUSTRIAL APPLICABILITY

According to the present invention, there are provided a therapeutic agent, a prophylactic agent, a food, a beverage and a feed for treatment or prevention of a disease requiring enhancement of growth factor production, comprising as an effective ingredient a polyphenol, a derivative thereof and/or a salt thereof; or a composition obtained by subjecting a polyphenol, a derivative thereof and/or a salt thereof to (i) a mixing treatment with a metal, a metal salt and/or a metal ion, or (ii) an oxidation treatment. The above-mentioned effective ingredient is especially excellent in enhancing actions for production of hepatocyte growth factor or a nerve growth factor. Therefore, according to a preferred embodiment of the present invention, there are provided a therapeutic agent, a prophylactic agent, a food, a beverage and a feed for treatment or prevention of a disease requiring enhancement for production of hepatocyte growth factor or a nerve growth factor.

## Claims

1. A therapeutic agent or prophylactic agent, **characterized in that** the agent comprises as an effective ingredient:
(i) at least one kind selected from the group consisting of polyphenols, derivatives thereof, and salts thereof; or
(ii) a composition obtained by subjecting at least one kind selected from the group consisting of polyphenols, derivatives thereof, and salts thereof to:
(A) a mixing treatment with at least one kind selected from the group consisting of a metal, a metal salt and a metal ion, or
(B) an oxidation treatment.

2. The therapeutic agent or prophylactic agent according to claim 1, wherein the polyphenol is at least one kind selected from the group consisting of flavonoids, gallic acid, chlorogenic acid, cryptochlorogenic acid, neochlorogenic acid, caffeic acid and dicaffeoylquinic acid.

3. The therapeutic agent or prophylactic agent according to claim 2, wherein the flavonoid is at least one kind selected from the group consisting of flavonols, flavanones, chalcones and flavanols.

4. The therapeutic agent or prophylactic agent according to claim 3, wherein the flavonol is myricetin and/or quercetin, the flavanone is isoxanthohumol, the chalcone is xanthohumol B and/or xanthohumol D, and the flavanol is epigallocatechin gallate.

5. The therapeutic agent or prophylactic agent according to any one of claims 1 to 4, wherein the derivative of a polyphenol is a carboxylic acid ester of a polyphenol and/or a glycoside of a polyphenol.

6. The therapeutic agent or prophylactic agent according to claim 5, wherein the carboxylic acid ester of a polyphenol is caffeic acid methyl ester and/or caffeic acid ethyl ester, and the glycoside of a polyphenol is isoorientin.

7. The therapeutic agent or prophylactic agent according to any one of claims 1 to 6, wherein the metal is at least one selected from the group consisting of iron, manganese, magnesium, copper, zinc, silver, gold, aluminum, calcium, nickel and cobalt, the metal salt is a salt containing the above-mentioned metal, and the metal ion is an ion of the above-mentioned metal.

8. The therapeutic agent or prophylactic agent according to any one of claims 1 to 7, wherein the growth factor is a hepatocyte growth factor or a nerve growth factor.

9. A food, beverage or feed, **characterized in that** the food, beverage or feed comprises as an effective ingredient:
(i) at least one kind selected from the group consisting of polyphenols, derivatives thereof, and salts thereof; or
(ii) a composition obtained by subjecting at least one kind selected from the group consisting of polyphenols, derivatives thereof, and salts thereof to:
(A) a mixing treatment with at least one kind selected from the group consisting of a metal, a metal salt and a metal ion, or
(B) an oxidation treatment.

10. The food, beverage or feed according to claim 9, wherein the polyphenol is at least one kind selected from the group consisting of flavonoids, gallic acid, chlorogenic acid, cryptochlorogenic acid, neochlorogenic acid, caffeic acid and dicaffeoylquinic acid.

11. The food, beverage or feed according to claim 10, wherein the flavonoid is at least one kind selected from the group consisting of flavonols, flavanones, chalcones and flavanols.

12. The food, beverage or feed according to claim 11, wherein the flavonol is myricetin and/or quercetin, the flavanone is isoxanthohumol, the chalcone is xanthohumol B and/or xanthohumol D, and the flavanol is epigallocatechin gallate.

13. The food, beverage or feed according to any one of claims 9 to 12, wherein the derivative of a polyphenol is a carboxylic acid ester of a polyphenol and/or a glycoside of a polyphenol.

14. The food, beverage or feed according to claim 13, wherein the carboxylic acid ester of a polyphenol is caffeic acid methyl ester and/or caffeic acid ethyl ester, and the glycoside of a polyphenol is isoorientin.

15. The food, beverage or feed according to any one of claims 9 to 14, wherein the metal is at least one selected from the group consisting of iron, manganese, magnesium, copper, zinc, silver, gold, aluminum, calcium, nickel and cobalt, the metal salt is a salt containing the above-mentioned metal, and the metal ion is an ion of the above-mentioned metal.

16. The food, beverage or feed according to any one of claims 9 to 15, wherein the growth factor is a hepatocyte growth factor or a nerve growth factor.

17. A composition obtained by subjecting at least one kind selected from the group consisting of polyphenols, derivatives thereof, and salts thereof to:
(A) a mixing treatment with at least one kind selected from the group consisting of a metal, a metal salt and a metal ion, or
(B) an oxidation treatment.

18. The composition according to claim 17, wherein the polyphenol is chlorogenic acid and/or caffeic acid, and the derivative of a polyphenol is a carboxylic acid ester of the above-mentioned polyphenol and/or a glycoside of the above-mentioned polyphenol.

19. The composition according to claim 17 or 18, wherein the metal is at least one selected from the group consisting of iron, manganese, magnesium, copper, zinc, silver, gold, aluminum, calcium, nickel and cobalt, the metal salt is a salt containing the above-mentioned metal, and the metal ion is an ion of the above-mentioned metal.

20. The composition according to any one of claims 17 to 19, which has enhancing action for growth factor production.

21. The composition according to claim 20, wherein the growth factor is a hepatocyte growth factor or a nerve growth factor.
